# EUROPEAN PATENT SPECIFICATION

(11) **EP 3 893 834 B1**
(45) Date of publication and mention of the grant of the patent: **29.04.2026**
(21) Application number: 19836607.2
(22) Date of filing: 06.12.2019
(51) Int. Cl.: A61K 8/365, A61K 8/46, A61Q 5/04, A61Q 5/06, A61K 8/44

(54) **STRAIGHTENING PROCESS FOR KERATIN FIBERS**
VERFAHREN ZUM GLÄTTEN VON KERATINFASERN
PROCÉDÉ DE LISSAGE DES FIBRES DE KÉRATINE

(30) Priority: 14.12.2018 JP 2018234350
(43) Date of publication of application: 20.10.2021
(73) Proprietor: L'OREAL, 75008 Paris (FR); Komure, Natsumi, Kawasaki-shi, Kanagawa 213-0012 (JP); Tsuzuki, Saki, Kanagawa, 2130012 (JP)
(72) Inventor: KOMURE Natsumi, Kawasaki-shi, Kanagawa 213-0012 (JP); TSUZUKI Saki, Kawasaki-shi, Kanagawa 213-0012 (JP)
(74) Representative: Lavoix
(86) International application number: PCT/JP2019/048836
(87) International publication number: WO 2020/122221

(56) References cited:
- WO-A1-2016/182086
- WO-A1-2016/182087
- CH-A2- 711 252
- US-A1- 2010 111 885
- US-A1- 2018 280 270

## Description

### TECHNICAL FIELD

The present invention relates to a process, in particular a cosmetic process, for keratin fibers such as hair.

### BACKGROUND ART

Straightening keratin fibers such as hair by heating the keratin fibers with an iron is popular for making unruly keratin fibers manageable and making keratin fibers have less volume and look more desirable. Generally, the instructions for such an iron mention that it should be used on dry keratin fibers to avoid vapor explosion and damage to the keratin fibers. Such a straightening method using a heating iron is convenient for quick style making, but the style lasts only short period of time, e.g., until the next shampooing. A long-lasting straightening method is required for many consumers.

On the other hand, conventional permanent straightening methods using a reducing agent and an alkaline agent, with heating, can provide keratin fibers with a long-lasting style, but they have several drawbacks such as long processing time, damage to the keratin fibers and malodor. Also, these methods usually require heating dry keratin fibers at 100-250 °C with an iron because vapor explosion can occur if the keratin fibers are wet. Therefore, these methods need a drying step before applying the iron onto the keratin fibers which takes time.

WO 2016/182086 A1 discloses a composition for reshaping keratin fibers with heat, comprising an alkylaminosulfonic acid in an amount of 8% by weight or more relative to the total weight of the composition, wherein the composition has a pH of from 8 to 12. US 2010/111885 A1 discloses a hair shape-controlling composition comprising a first component containing a reducing agent and an alkaline agent, and a second component containing an oxidizing agent, wherein the composition comprises a hydroxy-ether-amine compound or a quaternary ammonium salt thereof.

### DISCLOSURE OF INVENTION

An objective of the present invention is to provide a process, in particular a cosmetic process, for straightening keratin fibers such as hair, which can provide the keratin fibers with sufficient straightening effects which can last long time, as well as conditioning effects (e.g., providing the keratin fibers with softness and smoothness), with less damage than conventional straightening processes, and good usability (e.g., short processing time without drying before heating).

The above objective of the present invention can be achieved by a process for straightening keratin fibers, preferably hair, comprising the steps of:
(i) applying onto the keratin fibers a composition for straightening keratin fibers;
(ii) wrapping the keratin fibers, onto which the composition is applied, with at least one wrapping means in order to keep the keratin fibers wet;
(iii) straightening the wet keratin fibers with a heating iron at a temperature of more than 100 °C, preferably more than 100 °C and less than 210 °C, and more preferably from 110 °C to 200 °C;
(iv) unwrapping the keratin fibers; and
(v) optionally rinsing and/or drying the keratin fibers,
wherein
the composition for straightening keratin fibers comprises
(a) at least one compound chosen from alkylaminosulfonic acids and compounds of the following formulae (I) and (II): 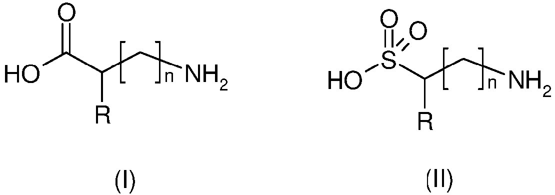 in which in formulae (I) and (II)
   - R represents a hydrogen atom, or a linear or branched, preferably linear, C₁-C₅ alkyl group, said alkyl group being optionally substituted with at least one group chosen from a hydroxyl group, an amino group, a carboxamido group, a C₆-C₁₈ aromatic group, a heterocyclic group,
   - C(O)-OH, -S(O)₂-OH, -C(O)-O⁻M⁺, -S(O)₂-O⁻M⁺, and mixtures thereof with M⁺ representing a cationic counterion such as an alkali metal, alkaline-earth metal, or ammonium, and
   - n is 0 or 1,
   and
b) monoethanolamine, and
the composition for straightening keratin fibers has a pH of from 7.5 to 12.0, preferably from 8.0 to 11.5, and more preferably from 8.5 to 11.0.

It is preferable that the wrapping means be heat-resistant, more preferably resistant to a temperature of more than 100 °C, even more preferably 110 °C or more, and even more preferably 120 °C or more.

It is preferable that the wrapping means form at least one occlusive space including the keratin fibers.

It may be preferable that the pH of the composition for straightening keratin fibers be within ±2 relative to a pH which is equal to the pKa of the following equilibrium:

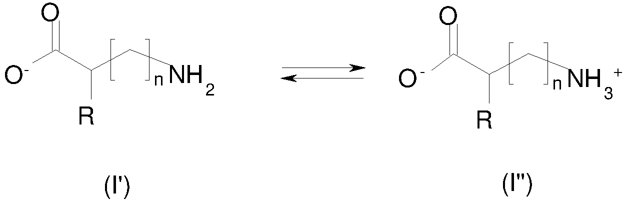

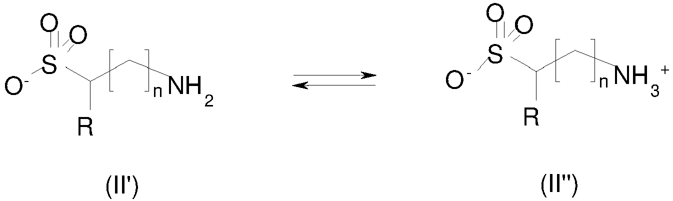

It may be preferable that the (a) compound be selected from the group consisting of alkylaminosulfonic acids such as 2-(cyclohexylamino)ethanesulfonic acid; amino acids such as glycine, alanine, glutamic acid, aspartic acid, phenylalanine, β-alanine, isoleucine, leucine, proline, glutamine, serine, threonine, valine, tryptophan, and tyrosine; oligomers of amino acids such as glycylglycine; aminosulfonic acids such as taurine; and mixtures thereof.

It may be more preferable that the (a) compound be selected from the group consisting of 2-(cyclohexylamino)ethanesulfonic acid, glycine, alanine, taurine, and mixtures thereof.

The amount of the (a) compound in the composition may be from 1% to 20% by weight, preferably from 3% to 15% by weight, and more preferably from 5% to 10% by weight, relative to the total weight of the composition.

The amount of the (b) monoethanolamine in the composition for straightening keratin fibers may be from 0.1% to 15% by weight, preferably from 0.5% to 10% by weight, and more preferably from 1% to 5% by weight, relative to the total weight of the composition.

The composition for straightening keratin fibers may comprise (c) at least one diol selected from C₄₋₅ diols.

The composition for straightening keratin fibers may comprise (d) at least one organic salt of alkaline earth metal.

It may be preferable that the composition for straightening keratin fibers does not comprise any ammonia or a thiol compound, or comprises less than 1%, preferably less than 0.5%, and more preferably less than 0.1% by weight of ammonia or a thiol compound, relative to the total weight of the composition.

It may be preferable that the composition for straightening keratin fibers does not comprise any reducing agent or oxidizing agent, or comprises less than 1%, preferably less than 0.5%, and more preferably less than 0.1% by weight of a reducing agent or an oxidizing agent, relative to the total weight of the composition.

### BEST MODE FOR CARRYING OUT THE INVENTION

After diligent research, the inventors have found that it is possible to provide a process, in particular a cosmetic process, for straightening keratin fibers such as hair, which can provide the keratin fibers with sufficient straightening effects which can last long time, as well as conditioning effects (e.g., providing the keratin fibers with softness and smoothness), with less damage than conventional straightening processes, and good usability (e.g., short processing time without drying before heating).

Thus, the present invention relates to a process for straightening keratin fibers, preferably hair, comprising the steps of:
(i) applying onto the keratin fibers a composition for straightening keratin fibers;
(ii) wrapping the keratin fibers, onto which the composition is applied, with at least one wrapping means in order to keep the keratin fibers wet;
(iii) straightening the wet keratin fibers with a heating iron at a temperature of more than 100 °C, preferably more than 100 °C and less than 210 °C, and more preferably from 110 °C to 200 °C;
(iv) unwrapping the keratin fibers; and
(v) optionally rinsing and/or drying the keratin fibers,
wherein
the composition for straightening keratin fibers comprises
(a) at least one compound chosen from alkylaminosulfonic acids and compounds of the following formula (I) and (II): 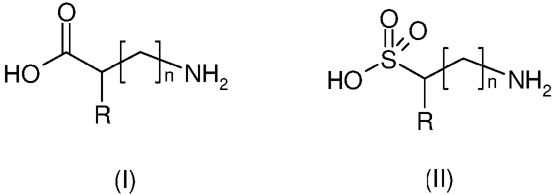 in which in formulae (I) and (II)
   - R represents a hydrogen atom, or a linear or branched, preferably linear, C₁-C₅ alkyl group, said alkyl group being optionally substituted with at least one group chosen from a hydroxyl group, an amino group, a carboxamido group, a C₆-C₁₈ aromatic group, a heterocyclic group,
   - C(O)-OH, -S(O)₂-OH, -C(O)-O⁻M⁺, -S(O)₂-O⁻M⁺, and mixtures thereof with M⁺ representing a cationic counterion such as an alkali metal, alkaline-earth metal, or ammonium, and
   - n is 0 or 1,
   and
b) monoethanolamine, andthe composition for straightening keratin fibers has a pH of from 7.5 to 12.0, preferably from 8.0 to 11.5, and more preferably from 8.5 to 11.0.

The process according to the present invention can provide keratin fibers with sufficient immediate straightening effects. Thus, the keratin fibers treated with the process according to the present invention can have less volume due to the reduction of curls of the keratin fibers. The straightening effects provided by the process according to the present invention can last a long time, for example, even after shampooing the keratin fibers 10 times.

The process according to the present invention can also provide keratin fibers with conditioning effects (e.g., providing the keratin fibers with softness and smoothness). Also, keratin fibers such as hair treated by the process according to the present invention can have good looking such as healthy and/or shiny appearance.

The process according to the present invention can provide keratin fibers with less damage as compared to conventional straightening processes. Therefore, it can be easy to comb the keratin fibers treated by the process according to the present invention. Thus, the keratin fibers treated with the process according to the present invention are easy to manage.

Also, the keratin fibers treated by the process according to the present invention can be stronger than those treated by conventional straightening processes.

The process according to the present invention can have good usability, for example, short processing time, no need for drying keratin fibers before heating, no malodor if no ammonia is used, and no need to use any reducing agent or oxidizing agent.

Hereafter, the process according to the present invention will be described in a detailed manner.

### [Process]

The present invention is a process for straightening keratin fibers, preferably hair, comprising the steps of:
(i) applying onto the keratin fibers a composition for straightening keratin fibers;
(ii) wrapping the keratin fibers, onto which the composition is applied, with at least one wrapping means in order to keep the keratin fibers wet;
(iii) straightening the wet keratin fibers with a heating iron at a temperature of more than 100 °C, preferably more than 100 °C and less than 210 °C, and more preferably from 110 °C to 200 °C;
(iv) unwrapping the keratin fibers; and
(v) optionally rinsing and/or drying the keratin fibers,
wherein
the composition for straightening keratin fibers comprises
(a) at least one compound chosen from alkylaminosulfonic acids and compounds of the following formulae (I) and (II): 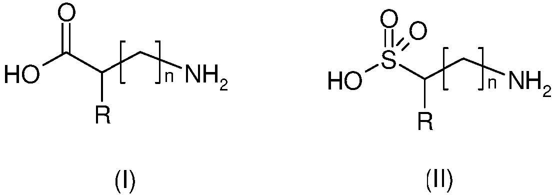 in which in formulae (I) and (II)
   - R represents a hydrogen atom, or a linear or branched, preferably linear, C₁-C₅ alkyl group, said alkyl group being optionally substituted with at least one group chosen from a hydroxyl group, an amino group, a carboxamido group, a C₆-C₁₈ aromatic group, a heterocyclic group,
   - C(O)-OH, -S(O)₂-OH, -C(O)-O⁻M⁺, -S(O)₂-O⁻M⁺, and mixtures thereof with M⁺ representing a cationic counterion such as an alkali metal, alkaline-earth metal, or ammonium, and
   - n is 0 or 1,
   and
b) monoethanolamine, and
the composition for straightening keratin fibers has a pH of from 7.5 to 12.0, preferably from 8.0 to 11.5, and more preferably from 8.5 to 11.0.

The details of the composition used in the process according to the present invention will be explained in the section titled [Composition] below.

The process according to the present invention is for the purpose of straightening keratin fibers such as hair, preferably straightening for a long period of time, and more preferably straightening even after 10 times or more of shampooing. In this sense, the process according to the present invention can be a permanent straightening process.

In step (i), the composition, which will be described later, is applied to keratin fibers. The application of the composition may be performed by any means, such as a brush and a comb.

The bath ratio of the applied composition to the keratin fibers may range from 0.1 to 10, more particularly from 0.5 to 5, and preferably between 0.8 and 1.2. The term "bath ratio" is intended to mean the weight ratio between the total weight of the applied composition and the total weight of the keratin fibers.

It may be possible that, after the application of the composition, the keratin fibers be left as they are for a certain amount of time; typically from 1 minute to 1 hour, preferably from 2 to 30 minutes, and more preferably from 3 to 10 minutes, if necessary, in order to let the composition penetrate into the keratin fibers.

In step (ii), the keratin fibers, onto which the composition is applied, are wrapped with at least one wrapping means in order to keep the keratin fibers wet. The keratin fibers are wrapped such that they remain wet during step (iii) which is explained below.

The wrapping means may be rigid or flexible. The wrapping means may comprise at least one member selected from the group consisting of a film and a sheet. The material of the film or the sheet is not limited. For example, the film or the sheet may comprise a thermoplastic or thermosetting resin, a paper, a textile, a bonnet, a metal foil such as aluminum foil, and the like.

It is preferable that the wrapping means used in step (ii), which may be a film or a sheet, be heat-resistant, more preferably resistant to a temperature of more than 100 °C, even more preferably 110 °C or more, and even more preferably 120 °C or more. It is also preferable that the wrapping means used in step (ii), which may be a film or a sheet, has high thermal conductivity and can prevent moisture evaporation. Therefore, it is preferable that the wrapping means comprise a metal foil such as aluminum foil.

It is preferable that the wrapping means, such as a film or a sheet, can form at least one occlusive space including the keratin fibers.

The occlusive space can restrict the evaporation of evaporable components such as water in the composition which has been applied to keratin fibers, and therefore, the temperature of the keratin fibers can be increased higher than that obtainable by a conventional heating process or device for the keratin fibers in open conditions. Furthermore, the keratin fibers can be heated effectively, and the keratin fibers can be heated evenly.

The occlusive space may form a condensation cage in which water and a component or components in the composition used in the process according to the present invention may evaporate from the keratin fibers, adhere to the wall of the film or sheet, and drop onto the keratin fibers. This cycle may be repeated during step (iii), i.e., heating the keratin fibers. Thus, the keratin fibers can always be kept wet, and drying and deterioration of the keratin fibers can be prevented.

The formation of the occlusive space may be preferable because the keratin fibers in the occlusive space can be kept wet and the temperature of the keratin fibers can be kept constant. The wet conditions of the keratin fibers may be preferable for the ingredients in the composition used in the process according to the present invention to effectively penetrate into the keratin fibers.

According to one variation of the present invention, the occlusive space may comprise apertures, the surface area of which is less than 5%, preferably less than 3% and more particularly less than 0.5% of the total surface area of the coating means. According to this variation, the total surface area of the coating means comprises the surface area of, when it is present, an opening means for the coating means.

The apertures may be passages, holes or orifices, which may allow an exchange of air between the occlusive space and the exterior thereof, especially if the reaction, such as the formation of vapor inside the occlusive space, is too great. On the other hand, a person skilled in the art could form the apertures such that the diffusion of heat in the occlusive space is not impaired.

In step (iii), the keratin fibers, which is wet, is subjected to straightening with a heating iron at a temperature of more than 100 °C.

As the heating iron, any conventional one can be used. The heating iron can have at least one plate, preferably two plates, which can be heated by, for example, electric heating. The heated plate(s) can be applied onto the wrapped keratin fibers and moved along with the direction of the keratin fibers to straighten them.

It is preferable that the heating iron has two plates, and that keratin fibers are sandwiched with the two plates, in which at least one of the plates can be heated, of the heating iron, and then the two plates are moved along with the direction of the keratin fibers to straighten them.

It is preferable that the heating iron, in particular a part thereof contacting with keratin fibers, such as a heating plate or heating plates, have a temperature of more than 100 °C and less than 210 °C, and more preferably from 110 °C to 200 °C. It is preferable that the keratin fibers be straightened at a temperature of more than 100 °C and less than 210 °C, and more preferably from 110 °C to 200 °C.

It may also be preferable that the heating iron, in particular a part thereof contacting with keratin fibers, such as a heating plate or heating plates, have a temperature of more than 100 °C and less than 190 °C, and more preferably more than 100 °C and less than 180 °C. It may be preferable that the keratin fibers be straightened at a temperature of more than 100 °C and less than 190 °C, and more preferably from 100 °C to 180 °C.

Step (iii) can be performed by one or more strokes of moving the heating iron along with the direction of keratin fibers. Step (iii) can be controlled by not only the temperature of the heating iron but also the number of strokes of heating iron.

The heating time may depend on the temperature of the heating iron but also the number of strokes of heating iron. It may be, for example, from 1 to 10 minutes, and preferably from 1 to 5 minutes.

In step (iv), the wrapped keratin fibers are unwrapped to expose the keratin fibers from the wrapping means.

In step (v), the keratin fibers may be rinsed preferably with water, and/or may be dried. The drying of the keratin fibers can be performed with a conventional drying means such as a hair drier.

### [Composition]

The composition for straightening keratin fibers used in the process according to the present invention comprises (a) at least one compound chosen from alkylaminosulfonic acids and compounds of the following formulae (I) and (II): 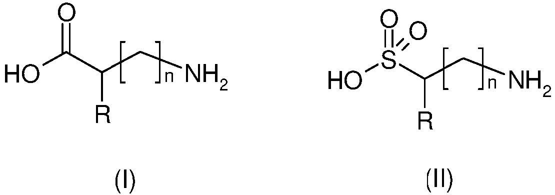 in which formulae (I) and (II)
- R represents a hydrogen atom, or a linear or branched, preferably linear, C₁-C₅ alkyl group, said alkyl group being optionally substituted with at least one group chosen from a hydroxyl group, an amino group, a carboxamido group, a C₆-C₁₈ aromatic group, a heterocyclic group, -C(O)-OH, -S(O)₂-OH, -C(O)-O⁻M⁺, -S(O)₂-O⁻M⁺, and mixtures thereof with M⁺ representing a cationic counterion such as an alkali metal, alkaline-earth metal, or ammonium, and
- n is 0 or 1,
and
b) monoethanolamine, and
the composition for straightening keratin fibers has a pH of from 7.5 to 12.0, preferably from 8.0 to 11.5, and more preferably from 8.5 to 11.0.

It is preferable that the above composition be a cosmetic composition, more preferably a cosmetic composition for straightening keratin fibers, and even more preferably a cosmetic composition for one-step straightening keratin fibers. It is particularly preferable that the keratin fibers be hair.

### (Active Compound)

The composition for straightening keratin fibers used in the process according to the present invention comprises (a) at least one compound chosen from alkylaminosulfonic acids and compounds of the above formulae (I) and (II), as active ingredient(s) for the hot straightening process for keratin fibers. Two or more of the (a) compounds may be used in combination. Thus, a single type of the (a) compound or a combination of different types of the (a) compounds may be used.

The alkylaminosulfonic acid may preferably have a C₁-C₂₀ alkyl group, preferably C₅-C₁₆ cycloalkyl group, and more preferably a C₆-C₁₂ cycloalkyl group, bonded to an imino group (-NH-) and a sulfonic acid moiety. The alkylaminosulfonic acid may be selected from the group consisting of 4-(2-hydroxyethyl)-1-piperazineethanesulfonic acid, 2-(cyclohexylamino)ethanesulfonic acid, and mixtures thereof.

The compound(s) of the above formulae (I) and (II) may be in their non-ionized form (I) or (II) or in their ionized or betaine form (I') or (II'): 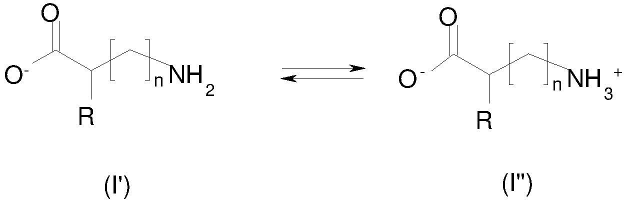 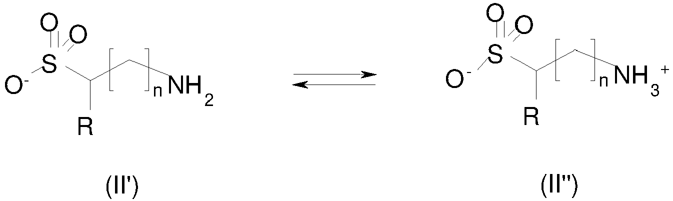 wherein
- R represents a hydrogen atom, or a linear or branched, preferably linear, C₁-C₅ alkyl group, said alkyl group being optionally substituted with at least one group chosen from a hydroxyl group, an amino group, a carboxamido group, a C₆-C₁₈ aromatic group, a heterocyclic group, -C(O)-OH, -S(O)₂-OH, -C(O)-O⁻M⁺, -S(O)₂-O⁻M⁺, and mixtures thereof with M⁺ representing a cationic counterion such as an alkali metal, alkaline-earth metal, or ammonium, and
- n is 0 or 1.

As the C₁-C₅ alkyl group, mention may be made of a methyl group, an ethyl group, a propyl group, an isopropyl group, an n-butyl group, a sec-butyl group, an iso-butyl group, a tert-butyl group and a pentyl group. A methyl group and an ethyl group are preferable, and a methyl group is more preferable.

As the amino group, mention may be made of -NH₂, a group including -NH₂ such as a sulfonylamino group, and a group including -NH-R' (wherein R' denotes a hydroxyl group or a C₁-C₅ alkyl group as mentioned above) such as a hydroxyamino group and a C₁-C₅ alkylamino group. It should be noted that the term "amino" group here does not mean a part of a urea group. As the amino group, -NH₂ is preferable.

As the C₆-C₁₈ aromatic group, mention may be made of a monovalent C₆-C₁₈ aryl group such as a phenyl group and a substituted phenyl group such as a hydroxyphenyl group and an aminophenyl group, and a monovalent C₇-C₁₈ aralkyl group such as a tolyl group.

As the heterocyclic group, mention may be made of a monovalent, saturated or unsaturated, substituted or unsubstituted heterocyclic group, such as a substituted or unsubstituted pyrrolyl group, a substituted or unsubstituted pyrrolidinyl group, a substituted or unsubstituted pyridyl group, a substituted or unsubstituted piperidono group, a substituted or unsubstituted piperidinyl group, a substituted or unsubstituted morpholino group, a substituted or unsubstituted morpholinyl group, a substituted or unsubstituted furyl group, and a substituted or unsubstituted indolyl group such as 3-indolyl group.

The compounds of the above formulae (I) and (II) correspond to amino acids and aminosulfonic acids, respectively.

The (a) compound(s) is(are) preferably chosen from "neutral" or "acidic" amino acids or aminosulfonic acids. The term "neutral" is intended to mean amino acids or aminosulfonic acids which have a pH, at ambient temperature (25°C), in water of inclusively between 5 and 7. The term "acidic" is intended to mean amino or aminosulfonic acids which have a pH, at ambient temperature, in water of less than 6.

Preferably, the amino acids or aminosulfonic acids may comprise a number of amino groups less than or equal to the number of acid groups.

The (a) compound(s) may be selected from the group consisting of alkylaminosulfonic acids such as 2-(cyclohexylamino)ethanesulfonic acid; amino acids such as glycine, alanine, glutamic acid, aspartic acid, phenylalanine, β-alanine, isoleucine, leucine, proline, glutamine, serine, threonine, valine, tryptophan, and tyrosine; oligomers of amino acids such as glycylglycine; aminosulfonic acids such as taurine; and mixtures thereof.

It is preferable that the (a) compound be selected from the group consisting of 2-(cyclohexylamino)ethanesulfonic acid, glycine, alanine, taurine, and mixtures thereof.

The amount of the (a) compound(s) in the composition used for the process according to the present invention may be 1% by weight or more, preferably 3% by weight or more, and more preferably 5% by weight or more, relative to the total weight of the composition.

On the other hand, the amount of the (a) compound(s) in the composition according to the present invention may be 20% by weight or less, preferably 15% by weight or less, and more preferably 10% by weight or less, relative to the total weight of the composition.

The amount of the (a) compound(s) in the composition used for the process according to the present invention may be from 1% to 20% by weight, preferably from 3% to 15% by weight, and more preferably from 5% to 10% by weight, relative to the total weight of the composition.

### (Alkaline Agent)

The composition for straightening keratin fibers used in the process according to the present invention comprises (b) at least one alkaline agent, wherein the at least one alkaline agent comprises monoethanolamine. Two or more (b) alkaline agents may be used in combination. Thus, monoethanolamine may be used as the only alkaline agent or a combination of monoethanolamine with different type(s) of alkaline agents may be used.

The (b) alkaline agent is different from the (a) compound.

The composition according to the present invention include (b) alkaline agent(s), if the (a) compound can function to reduce the pH of the composition.

In addition to monoethanolamine, the (b) alkaline agent may further comprise an inorganic alkaline agent. It is preferable that the (b) alkaline agent be non-volatile. It is preferable that the inorganic alkaline agent be selected from the group consisting of alkaline metal hydroxides; alkaline earth metal hydroxides; and alkaline metal phosphates and monohydrogen phosphates such as sodium phosphate or sodium monohydrogen phosphate.

As examples of the inorganic alkaline metal hydroxides, mention may be made of sodium hydroxide, lithium hydroxide and potassium hydroxide. As examples of the alkaline earth metal hydroxides, mention may be made of calcium hydroxide and magnesium hydroxide. As the inorganic alkaline agent, sodium hydroxide and potassium hydroxide are preferable.

In addition to monoethanolamine, the (b) alkaline agent may comprise a further organic alkaline agent. It is preferable that the further organic alkaline agent be selected from the group consisting of basic amino acids, monoamines and diamines.

The basic amino acid comprises an additional amine function optionally included in a ring or in a ureido function. Such basic amino acids may be preferably chosen from those corresponding to formula (A) below: 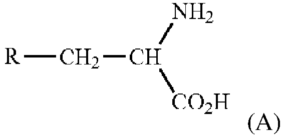 in which R denotes a group chosen from:

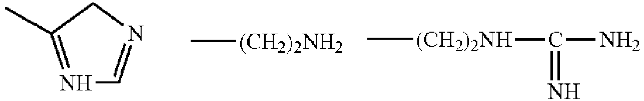

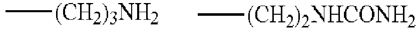

The compounds corresponding to formula (A) may be histidine, lysine, arginine, ornithine and citrulline

As examples of the monoamines, mention may be made of alkanolamines such as mono-, di- and tri-ethanolamine, comprising 1 to 3 hydroxyalkyl(C₁-C₄) groups. Particularly, alkanolamines may be selected from diethanolamine, triethanolamine, monoisopropanolamine, diisopropanolamine, N,N-dimethylethanolamine, 2-amino-2-methyl-1-propanol, triisopropanolamine, 2-amino-2-methyl-1,3-propanediol, 3-amino-1,2-propanediol, 3-dimethylamino-1,2-propanediol, and tris(hydroxymethylamino)methane.

The diamines may be described in the structure (B) below: 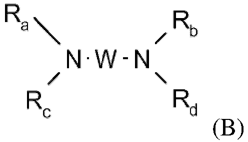 wherein W denotes an alkylene such as propylene optionally substituted by a hydroxyl or a C₁-C₄ alkyl radical, and Rₐ, R_{b}, R_{c} and R_{d} independently denote a hydrogen atom, an alkyl radical or a C₁-C₄ hydroxyalkyl radical, which may be exemplified by 1,3-propanediamine and derivatives thereof.

The amount of the (b) alkaline agent(s) in the composition used for the process according to the present invention may be 0.1% by weight or more, preferably 0.5% by weight or more, and more preferably 1% by weight or more, relative to the total weight of the composition.

On the other hand, the amount of the (a) alkaline agent(s) in the composition used for the process according to the present invention may be 15% by weight or less, preferably 10% by weight or less, and more preferably 5% by weight or less, relative to the total weight of the composition.

The (b) alkaline agent(s) may be used in a total amount of from 0.1% to 15% by weight, preferably from 0.5% to 10% by weight, and more preferably from 1% to 5% by weight, relative to the total weight of the composition.

### (Acid)

The composition for straightening keratin fibers used in the process according to the present invention may comprise at least one acid which is different from the (a) compound. Two or more acids may be used in combination. Thus, a single type of acid or a combination of different types of acids may be used.

The acid may be used to adjust the pH of the composition according to the present invention.

It is preferable that the composition according to the present invention include acid(s), if the (a) compound can function to increase the pH of the composition.

As the acid, mention may be made of any inorganic or organic acids which are commonly used in cosmetic products such as citric acid, lactic acid, sulfuric acid, phosphoric acid or hydrochloric acid (HCl). HCl is preferable.

The amount of the acid(s) in the composition used for the process according to the present invention may be 0.1% by weight or more, preferably 0.5% by weight or more, and more preferably 1% by weight or more, relative to the total weight of the composition.

On the other hand, the amount of the (a) alkaline agent(s) in the composition used for the process according to the present invention may be 15% by weight or less, preferably 10% by weight or less, and more preferably 5% by weight or less, relative to the total weight of the composition.

The acid(s) may be used in a total amount of from 0.1% to 15% by weight, preferably from 0.5% to 10% by weight, and more preferably from 1% to 5% by weight, relative to the total weight of the composition, depending on their solubility.

### (Diol)

The composition for straightening keratin fibers used in the process according to the present invention may comprise (c) at least one diol selected from C₄₋₅ diols. Thus, a single type of (c) diol or a combination of different types of (c) diols may be used.

Although not bound by any theory, it is believed that the (c) diol can loosen the hydrophobic interaction between keratin fibers to increase reshaping efficiency.

The C₄₋₅ diols can be butyleneglycol and pentyleneglycol.

Butyleneglycol encompasses isomers thereof. Thus, butyleneglycol may be, for example, 1,2-butyleneglycol, 1,3-butyleneglycol, 2,3-butyleneglycol and 1,4-butyleneglycol. 1,3-butyleneglycol may be preferable.

Pentyleneglycol encompasses isomers thereof. Thus, pentyleneglycol may be 1,2-pentyleneglycol, 1,3-pentyleneglycol, 1,4-pentyleneglycol, 1,5-pentyleneglycol, 2,3-pentyleneglycol, and 2,4-penyleneglycol. 1,2-pentyleneglycol may be preferable.

The amount of the (c) diol(s) in the composition according to the present invention may be 0.01% by weight or more, preferably 0.1% by weight or more, more preferably more than 1% by weight, and even more preferably more than 3% by weight, relative to the total weight of the composition.

On the other hand, the amount of the (a) diol(s) in the composition according to the present invention may be 20% by weight or less, preferably 15% by weight or less, and more preferably 10% by weight or less, relative to the total weight of the composition.

The amount of the (a) diol(s) in the composition according to the present invention may range from 0.01% to 20% by weight, preferably from 0.1% to 15% by weight, more preferably from more than 1% to 10% by weight, and even more preferably from more than 3% to 10% by weight, relative to the total weight of the composition.

### (Organic Salt of Alkaline Earth Metal)

The composition for straightening keratin fibers used in the process according to the present invention may comprise (d) at least one organic acid salt of alkaline earth metal. Two or more such salts may be used in combination. Thus, a single type of such salt or a combination of different types of such salts may be used.

If a plurality of organic acid salts of alkaline earth metal are used, it is possible that the type of organic acid is different and/or the type of alkaline earth metal is different.

The alkaline earth metal may be selected from magnesium and calcium.

The organic acid may be selected from α-hydroxy acids.

The α-hydroxy acids may be represented by the following formula (I): 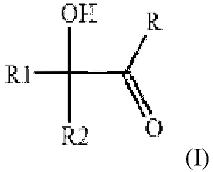 wherein
R1=H, -OH, -NH₂, -CH₂COOH or a linear or branched C₁₋₄ alkyl,
R2=H,- COOH, -CHOH-COOH, -CF₃,- CH=CH₂, -NHCONH₂, a linear, branched or cyclic C₁₋₈ alkyl optionally substituted with a radical chosen from -OH, Cl, -NH₂, -COOH, -CF₃ and
-SCH₃; a phenyl or benzyl optionally substituted with one -OH or -OCH₃ radical; or alternatively the radical 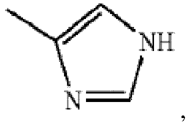 and
R1 and R2 may also together form an oxo radical (=O) or a cyclopropyl, cyclobutyl, hydroxycyclobutyl, cyclopentyl or cyclohexyl ring with the carbon atom that bears them, or alternatively the radical 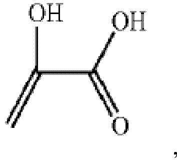 and
when R1=H, R2 may also represent a -(CHOH)₂CH₂OH or -(CHOH)₃CH₂OH radical, R = -OH or -NR3R4 with R3, R4 = H or a linear or branched C₁₋₄ alkyl optionally substituted with one or two OH radicals, as well as stereoisomers, organic or mineral salts and solvates thereof.

The α-hydroxy acids may be selected from the following:
glycolic acid, oxalic acid, lactic acid, 1-hydroxy-1-cyclopropanecarboxylic acid, 2-hydroxy-3-butenoic acid, 2-hydroxyisobutyric acid, 2-hydroxy-n-butyric acid, isoserine, glyceric acid, 2-hydroxy-3-methylbutyric acid, 2-hydroxy-2-methylbutyric acid, 2-hydroxyvaleric acid, 4-amino-2-hydroxybutyric acid, 1-hydroxycyclohexanecarboxylic acid, dihydroxyfumaric acid, citramalic acid, tartaric acid, citric acid, 2-hydroxy-4-(methylthio)butyric acid, mandelic acid, 2-hydroxy-3-methylvaleric acid, glyoxylurea, β-imidazolelactic acid, 2-trifluoromethyl-2-hydroxypropionic acid, hexahydromandelic acid, 2-hydroxyoctanoic acid, arabic acid, 3-phenylactic acid, hydroxyphenylglycine, 3-hydroxymandelic acid, 4-hydroxymandelic acid, 2-hydroxynonanoic acid, L-arginic acid, 3-methoxymandelic acid, 4-methoxymandelic acid, 3-(4-hydroxyphenyl)lactic acid, tartronic acid, β-chlorolactic acid, 1-cyclopentanol-1-carboxylic acid, 1,2-dihydroxycyclobutanecarboxylic acid, 2-ethyl-2-hydroxybutric acid, α-hydroxyisocaproic acid, α-hydroxycaproic acid, 2-hydroxy-3,3-dimethylbutyric acid, malic acid, hydroxytartronic acid, gluconic acid, lactamide, N-methyllactamide, N-ethyllactamide, N,N-dimethyllactamide, N-2-hydroxyethyllactamide, and stereoisomers, organic or mineral salts and solvates thereof.

It may be preferable that the α-hydroxy acid be selected from the group consisting of glycolic acid, oxalic acid, L-lactic acid, DL-lactic acid, D-lactic acid, malic acid, tartaric acid, DL-glyceric acid, arabic acid, gluconic acid, hydroxytartronic acid, lactamide, N-methyllactamide, N-ethyllactamide, and N-2-hydroxyethyllactamide.

It may be more preferable that α-hydroxy acid be selected from the group consisting of gluconic acid, glycolic acid, lactic acid, malic acid, citric acid, tartaric acid, and mandelic acid.

It may be even more preferable that the α-hydroxy acid be gluconic acid. If gluconate is used as the organic acid salt of alkaline earth metal, in particular Mg, the time period of heating step (ii) in the process according to the present invention can be shortened.

Although not bound by any theory, it is believed that this effect is based on catalytic effects of the alkaline earth metal gluconate such as magnesium gluconate.

The amount of the (d) organic acid salt(s) of alkaline earth metal in the composition according to the present invention may be 0.001% by weight or more, preferably 0.01% by weight or more, and more preferably more than 0.05% by weight, relative to the total weight of the composition.

On the other hand, the amount of the (d) organic acid salt(s) of alkaline earth metal in the composition according to the present invention may be 10% by weight or less, preferably 5% by weight or less, and more preferably 1% by weight or less, relative to the total weight of the composition.

The amount of the (d) organic acid salt(s) of alkaline earth metal in the composition may be from 0.001% to 10% by weight, preferably from 0.01% to 5% by weight, and more preferably from more than 0.05% to 1% by weight, relative to the total weight of the composition.

### (Oil)

The composition for straightening keratin fibers used in the process according to the present invention may further comprise at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils, and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, coconut oil, and mixtures thereof.

As examples of synthetic oils, mention may be made of alkane oils such as 2, 6, 10-trimethyldodecane (farnesane), isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C, and even more particularly from:
(i) cyclic polydialkylsiloxanes comprising from 3 to 7 and preferably 4 to 5 silicon atoms. These are, for example, octamethylcyclotetrasiloxane sold in particular under the name Volatile Silicone^{®} 7207 by Union Carbide or Silbione^{®} 70045 V2 by Rhodia, decamethylcyclopentasiloxane sold under the name Volatile Silicone^{®} 7158 by Union Carbide, Silbione^{®} 70045 V5 by Rhodia, and dodecamethylcyclopentasiloxane sold under the name Silsoft 1217 by Momentive Performance Materials, and mixtures thereof. Mention may also be made of cyclocopolymers of the type such as dimethylsiloxane/methylalkylsiloxane, such as Silicone Volatile^{®} FZ 3109 sold by the company Union Carbide, of formula:
   with D" : 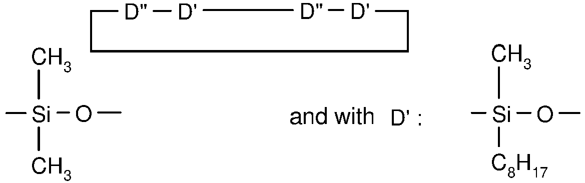 Mention may also be made of mixtures of cyclic polydialkylsiloxanes with organosilicon compounds, such as the mixture of octamethylcyclotetrasiloxane and tetratrimethylsilylpentaerythritol (50/50) and the mixture of octamethylcyclotetrasiloxane and oxy-1,1'-bis(2,2,2',2',3,3'-hexatrimethylsilyloxy)neopentane; and
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.* The viscosity of the silicones is measured at 25°C according to ASTM standard 445 Appendix C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Corning, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

The phenyl silicone oil may be chosen from the phenyl silicones of the following formula: 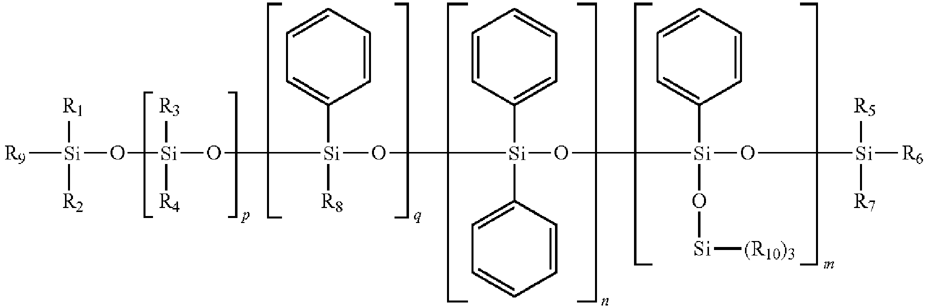 in which
R₁ to R₁₀, independently of each other, are saturated or unsaturated, linear, cyclic or branched C₁-C₃₀ hydrocarbon-based radicals, preferably C₁-C₁₂ hydrocarbon-based radicals, and more preferably C₁-C₆ hydrocarbon-based radicals, in particular methyl, ethyl, propyl, or butyl radicals, and
m, n, p, and q are, independently of each other, integers of 0 to 900 inclusive, preferably 0 to 500 inclusive, and more preferably 0 to 100 inclusive,
with the proviso that the sum n+m+q is other than 0.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone (R₁ to R₁₀ are methyl; p, q, and n = 0; m=1 in the above formula) is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane and hemisqualane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition for the process according to the present invention is preferably chosen from cetyl alcohol, octyldodecanol, hexyldecanol, and mixtures thereof.

It may be preferable that the oil be chosen from fatty alcohols such as cetearyl alcohol, alkane oils such as farnesane and silicone oils such as dimethicone.

The amount of the oil in the composition for the process according to the present invention may be 0.01% by weight or more, preferably 0.05% by weight or more, more preferably 0.1% by weight or more, and even more preferably 0.2% by weight or more, relative to the total weight of the composition.

On the other hand, the amount of the oil in the composition for the process according to the present invention may be 25% by weight or less, preferably 20% by weight or less, more preferably 15% by weight or less, and even more preferably 10% by weight or less, relative to the total weight of the composition.

The amount of the oil in the composition for the process according to the present invention may range from 0.01% to 25% by weight, preferably from 0.05% to 20% by weight, more preferably from 0.1% to 15% by weight, and even more preferably from 0.2% to 10% by weight, relative to the total weight of the composition.

### (Surfactant)

The composition for straightening keratin fibers used in the process according to the present invention may further comprise at least one surfactant. Two or more surfactants may be used. Thus, a single type of surfactant or a combination of different types of surfactants may be used.

Any surfactant may be used for the present invention. The surfactant may be selected from the group consisting of anionic surfactants, amphoteric surfactants, cationic surfactants and nonionic surfactants. Two or more surfactants may be used in combination. Thus, a single type of surfactant or a combination of different types of surfactants may be used.

According to one embodiment of the present invention, the amount of the surfactant(s) may range from 0.01 to 15% by weight, preferably from 0.05 to 10% by weight, and more preferably from 0.1 to 5% by weight, relative to the total weight of the composition used in the process according to the present invention.

### (i) Anionic Surfactants

The composition may comprise at least one anionic surfactant. Two or more anionic surfactants may be used in combination.

It is preferable that the anionic surfactant be selected from the group consisting of (C₆-C₃₀)alkyl sulfates, (C₆-C₃₀)alkyl ether sulfates, (C₆-C₃₀)alkylamido ether sulfates, alkylaryl polyether sulfates, monoglyceride sulfates; (C₆-C₃₀)alkylsulfonates, (C₆-C₃₀)alkylamide sulfonates, (C₆-C₃₀)alkylaryl sulfonates, α-olefin sulfonates, paraffin sulfonates; (C₆-C₃₀)alkyl phosphates; (C₆-C₃₀)alkyl sulfosuccinates, (C₆-C₃₀)alkyl ether sulfosuccinates, (C₆-C₃₀)alkylamide sulfosuccinates; (C₆-C₃₀)alkyl sulfoacetates; (C₆-C₂₄)acyl sarcosinates; (C₆-C₂₄)acyl glutamates; (C₆-C₃₀)alkylpolyglycoside carboxylic ethers; (C₆-C₃₀)alkylpolyglycoside sulfosuccinates; (C₆-C₃₀)alkyl sulfosuccinamates; (C₆-C₂₄)acyl isethionates; N-(C₆-C₂₄)acyl taurates; C₆-C₃₀ fatty acid salts; coconut oil acid salts or hydrogenated coconut oil acid salts; (C₈-C₂₀)acyl lactylates; (C₆-C₃₀)alkyl-D-galactoside uronic acid salts; polyoxyalkylenated (C₆-C₃₀)alkyl ether carboxylic acid salts; polyoxyalkylenated (C₆-C₃₀)alkylaryl ether carboxylic acid salts; and polyoxyalkylenated (C₆-C₃₀)alkylamido ether carboxylic acid salts; and corresponding acid forms.

In at least one embodiment, the anionic surfactants are in the form of salts such as salts of alkali metals, for instance sodium; salts of alkaline-earth metals, for instance magnesium; ammonium salts; amine salts; and amino alcohol salts. Depending on the conditions, they may also be in acid form.

It is more preferable that the anionic surfactant be selected from salts of (C₆-C₃₀)alkyl sulfate, (C₆-C₃₀)alkyl ether sulfate or polyoxyalkylenated (C₆-C₃₀)alkyl ether carboxylic acid, salified or not.

### (ii) Amphoteric Surfactants

The composition may comprise at least one amphoteric surfactant. Two or more amphoteric surfactants may be used in combination.

The amphoteric or zwitterionic surfactants can be, for example (non-limiting list), amine derivatives such as aliphatic secondary or tertiary amine, and optionally quaternized amine derivatives, in which the aliphatic radical is a linear or branched chain including 8 to 22 carbon atoms and containing at least one water-solubilizing anionic group (for example, carboxylate, sulphonate, sulphate, phosphate or phosphonate).

The amphoteric surfactant may preferably be selected from the group consisting of betaines and amidoaminecarboxylated derivatives.

It is preferable that the amphoteric surfactant be selected from betaine-type surfactants.

The betaine-type amphoteric surfactant is preferably selected from the group consisting of alkylbetaines, alkylamidoalkylbetaines, sulfobetaines, phosphobetaines, and alkylamidoalkylsulfobetaines, in particular, (C₈-C₂₄)alkylbetaines, (C₈-C₂₄)alkylamido(C₁-C₈)alkylbetaines, sulphobetaines, and (C₈-C₂₄)alkylamido(C₁-C₈)alkylsulphobetaines. In one embodiment, the amphoteric surfactants of betaine type are chosen from (C₈-C₂₄)alkylbetaines, (C₈-C₂₄)alkylamido(C₁-C₈)alkylsulphobetaines, sulphobetaines, and phosphobetaines.

Non-limiting examples that may be mentioned include the compounds classified in the CTFA International Cosmetic Ingredient Dictionary & Handbook, 15th Edition, 2014, under the names cocobetaine, laurylbetaine, cetylbetaine, coco/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, alone or as mixtures.

The betaine-type amphoteric surfactant is preferably an alkylbetaine and an alkylamidoalkylbetaine, in particular cocobetaine and cocamidopropylbetaine.

Among the amidoaminecarboxylated derivatives, mention may be made of the products sold under the name Miranol, as described in U.S. Pat. Nos. 2,528,378 and 2,781,354 and classified in the CTFA dictionary, 3rd edition, 1982 , under the names Amphocarboxyglycinates and Amphocarboxypropionates, with the respective structures:

R₁-CONHCH₂CH₂-N⁺(R₂)(R₃)(CH₂COO⁻) M⁺ X⁻ (B1)

in which:
R₁ denotes an alkyl radical of an acid R₁-COOH present in hydrolysed coconut oil, a heptyl, nonyl or undecyl radical,
R₂ denotes a beta-hydroxyethyl group,
R₃ denotes a carboxymethyl group,
M⁺ denotes a cationic ion derived from alkaline metals such as sodium; ammonium ion; or an ion derived from an organic amine;
X⁻ denotes an organic or inorganic anionic ion such as halides, acetates, phosphates, nitrates, alkyl(C₁-C₄)sulfates, alkyl(C₁-C₄)- or alkyl(C₁-C₄)aryl-sulfonates, particularly methylsulfate and ethylsulfate; or M⁺ and X⁻ are not present;

   R₁'-CONHCH₂CH₂-N(B)(C) (B2)

   in which:
   R₁' denotes an alkyl radical of an acid R₁'-COOH present in coconut oil or in hydrolysed linseed oil, an alkyl radical, such as a C₇, C₉, C₁₁ or C₁₃ alkyl radical, a C₁₇ alkyl radical and
   its iso-form, or an unsaturated C₁₇ radical,
   B represents -CH₂CH₂OX',
   C represents -(CH₂)_{z}-Y', with z=1 or 2,
   X' denotes a -CH₂-COOH group, -CH₂-COOZ', -CH₂CH₂-COOH, -CH₂CH₂-COOZ' or a hydrogen atom, and
   Y' denotes -COOH, -COOZ', -CH₂-CHOH-SO₃Z', a -CH₂-CHOH-SO₃H radical or a -CH₂-CH(OH)-SO₃-Z' radical,
   wherein Z' represents an ion of an alkaline or alkaline earth metal such as sodium, an ion derived from an organic amine or an ammonium ion;
   and

   R_{a"}-NH-CH(Y")-(CH₂)ₙ-C(O)-NH-(CH₂)_{n'}-N(Rd)(Re) (B'2)

   in which:
   Y" denotes -C(O)OH, -C(O)OZ", -CH₂-CH(OH)-SO₃H or -CH₂-CH(OH)-SO₃-Z", wherein Z" denotes a cationic ion derived from alkaline metal or alkaline-earth metals such as sodium, an ion derived from organic amine or an ammonium ion;
   Rd and Re denote a C₁-C₄ alkyl or C₁-C₄ hydroxyalkyl radical;
   R_{a"} denotes a C₁₀-C₃₀ group alkyl or alkenyl group from an acid, and
   n and n' independently denote an integer from 1 to 3.

It is preferable that the amphoteric surfactant with formula B1 and B2 be selected from (C₈-C₂₄)-alkyl amphomonoacetates, (C₈-C₂₄)alkyl amphodiacetates, (C₈-C₂₄)alkyl amphomonopropionates, and (C₈-C₂₄)alkyl amphodipropionates

These compounds are classified in the CTFA dictionary, 5th edition, 1993, under the names Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphopropionate, Disodium Caprylamphodipropionate, Disodium Caprylamphodipropionate, Lauroamphodipropionic acid and Cocoamphodipropionic acid.

By way of example, mention may be made of the cocoamphodiacetate sold under the trade name Miranol^{®} C2M concentrate by the company Rhodia Chimie.

Among compounds of formula (B'2) mention may be made of sodium diethylaminopropyl cocoaspartamide (CTFA) marketed by CHIMEX under the denomination CHIMEXANE HB.

### (iii) Cationic Surfactants

The composition may comprise at least one cationic surfactant. Two or more cationic surfactants may be used in combination.

The cationic surfactant may be selected from the group consisting of optionally polyoxyalkylenated, primary, secondary or tertiary fatty amine salts, quaternary ammonium salts, and mixtures thereof.

Examples of quaternary ammonium salts that may be mentioned include, but are not limited to:
those of general formula (B3) below: 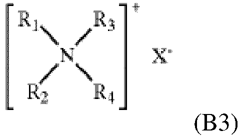
wherein
R₁, R₂, R₃, and R₄, which may be identical or different, are chosen from linear and branched aliphatic radicals including from 1 to 30 carbon atoms and optionally including heteroatoms such as oxygen, nitrogen, sulfur and halogens. The aliphatic radicals may be chosen, for example, from alkyl, alkoxy, C₂-C₆ polyoxyalkylene, alkylamide, (C₁₂-C₂₂)alkylamido(C₂-C₆)alkyl, (C₁₂-C₂₂)alkylacetate and hydroxyalkyl radicals; and aromatic radicals such as aryl and alkylaryl; and X⁻ is chosen from halides, phosphates, acetates, lactates, (C₂-C₆) alkyl sulfates and alkyl- or alkylaryl-sulfonates;
quaternary ammonium salts of imidazoline, for instance those of formula (B4) below: 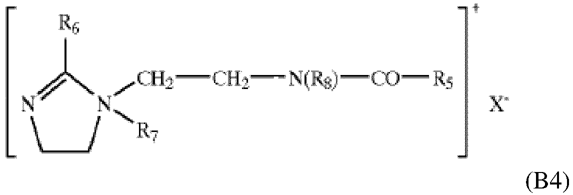 wherein:
   R₅ is chosen from alkenyl and alkyl radicals including from 8 to 30 carbon atoms, for example fatty acid derivatives of tallow or of coconut;
   R₆ is chosen from hydrogen, C₁-C₄ alkyl radicals, and alkenyl and alkyl radicals including from 8 to 30 carbon atoms;
   R₇ is chosen from C₁-C₄ alkyl radicals;
   R₈ is chosen from hydrogen and C₁-C₄ alkyl radicals; and
   X⁻ is chosen from halides, phosphates, acetates, lactates, alkyl sulfates, alkyl sulfonates, and alkylaryl sulfonates. In one embodiment, R₅ and R₆ are, for example, a mixture of radicals chosen from alkenyl and alkyl radicals including from 12 to 21 carbon atoms, such as fatty acid derivatives of tallow, R₇ is methyl and R₈ is hydrogen. Examples of such products include, but are not limited to, Quaternium-27 (CTFA 1997) and Quaternium-83 (CTFA 1997), which are sold under the names "Rewoquat^{®}" W75, W90, W75PG and W75HPG by the company Witco;
   di or tri quaternary ammonium salts of formula (B5): 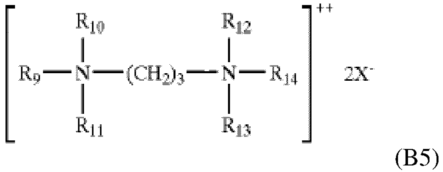 wherein:
      R₉ is chosen from aliphatic radicals including from 16 to 30 carbon atoms;
      R₁₀ is chosen from hydrogen or alkyl radicals including from 1 to 4 carbon atoms or a group -(CH2)3 (R₁₆ₐ)(R₁₇ₐ)(R₁₈ₐ)N⁺X-₋;
      R₁₁, R₁₂, R₁₃, R₁₄, R₁₆ₐ, R₁₇ₐ, and R₁₈ₐ, which may be identical or different, are chosen from hydrogen and alkyl radicals including from 1 to 4 carbon atoms; and
      X⁻ is chosen from halides, acetates, phosphates, nitrates, ethyl sulfates, and methyl sulfates.

An example of one such diquaternary ammonium salt is FINQUAT CT-P of FINETEX (Quaternium-89) or FINQUAT CT (Quaternium-75);
and
quaternary ammonium salts including at least one ester function, such as those of formula (B6) below: 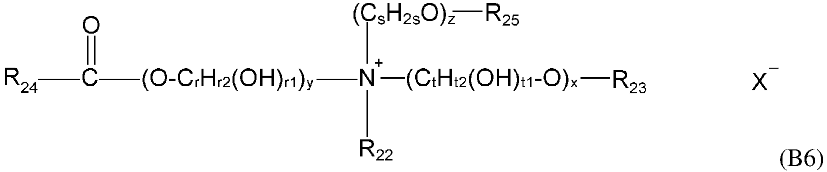 wherein:
R₂₂ is chosen from C₁-C₆ alkyl radicals and C₁-C₆ hydroxyalkyl and dihydroxyalkyl radicals;
R₂₃ is chosen from:
   the radical below: 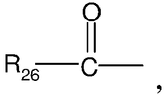
   linear and branched, saturated and unsaturated C₁-C₂₂ hydrocarbon-based radicals R₂₇, and hydrogen,
   R₂₅ is chosen from:
      the radical below: 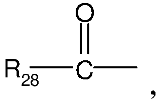
      linear and branched, saturated and unsaturated C₁-C₆ hydrocarbon-based radicals R₂₉, and hydrogen,
      R₂₄, R₂₆, and R₂₈, which may be identical or different, are chosen from linear and branched, saturated and unsaturated, C₇-C₂₁, hydrocarbon-based radicals;
      r, s, and t, which may be identical or different, are chosen from integers ranging from 2 to 6; each of r1 and t1, which may be identical or different, is 0 or 1, and r2+r1=2r and t1+2t=2t; y is chosen from integers ranging from 1 to 10;
      x and z, which may be identical or different, are chosen from integers ranging from 0 to 10;
      X⁻ is chosen from simple and complex, organic and inorganic anions; with the proviso that the sum x+y+z ranges from 1 to 15, that when x is 0, R₂₃ denotes R₂₇, and that when z is 0, R₂₅ denotes R₂₉. R₂₂ may be chosen from linear and branched alkyl radicals. In one embodiment, R₂₂ is chosen from linear alkyl radicals. In another embodiment, R₂₂ is chosen from methyl, ethyl, hydroxyethyl, and dihydroxypropyl radicals, for example methyl and ethyl radicals. In one embodiment, the sum x+y+z ranges from 1 to 10. When R₂₃ is a hydrocarbon-based radical R₂₇, it may be long and include from 12 to 22 carbon atoms, or short and include from 1 to 3 carbon atoms. When R₂₅ is a hydrocarbon-based radical R₂₉, it may include, for example, from 1 to 3 carbon atoms. By way of a non-limiting example, in one embodiment, R₂₄, R₂₆, and R₂₈, which may be identical or different, are chosen from linear and branched, saturated and unsaturated, C₁₁-C₂₁ hydrocarbon-based radicals, for example from linear and branched, saturated and unsaturated C₁₁-C₂₁ alkyl and alkenyl radicals. In another embodiment, x and z, which may be identical or different, are 0 or 1. In one embodiment, y is equal to 1. In another embodiment, r, s and t, which may be identical or different, are equal to 2 or 3, for example equal to 2. The anion X⁻ may be chosen from, for example, halides, such as chloride, bromide, and iodide; and C₁-C₄ alkyl sulfates, such as methyl sulfate. However, methane sulfonate, phosphate, nitrate, tosylate, an anion derived from an organic acid, such as acetate and lactate, and any other anion that is compatible with the ammonium including an ester function, are other non-limiting examples of anions that may be used according to the present invention. In one embodiment, the anion X⁻ is chosen from chloride and methyl sulfate.

In another embodiment, the ammonium salts of formula (B6) may be used, wherein:
R₂₂ is chosen from methyl and ethyl radicals,
x and y are equal to 1;
z is equal to 0 or 1;
r, s and t are equal to 2;
R₂₃ is chosen from:
   the radical below: 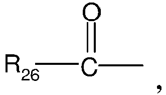
   methyl, ethyl, and C₁₄-C₂₂ hydrocarbon-based radicals, hydrogen;
   R₂₅ is chosen from:
      the radical below: 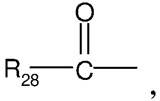
      and hydrogen;
      R₂₄, R₂₆, and R₂₈, which may be identical or different, are chosen from linear and branched, saturated and unsaturated, C₁₃-C₁₇ hydrocarbon-based radicals, for example from linear and branched, saturated and unsaturated, C₁₃-C₁₇ alkyl and alkenyl radicals.

In one embodiment, the hydrocarbon-based radicals are linear.

Non-limiting examples of compounds of formula (B6) that may be mentioned include salts, for example chloride and methyl sulfate, of diacyloxyethyl-dimethylammonium, of diacyloxyethyl-hydroxyethyl-methylammonium, of monoacyloxyethyl-dihydroxyethyl-methylammonium, of triacyloxyethyl-methylammonium, of monoacyloxyethyl-hydroxyethyl-dimethyl-ammonium, and mixtures thereof. In one embodiment, the acyl radicals may include from 14 to 18 carbon atoms, and may be derived, for example, from a plant oil, for instance palm oil and sunflower oil. When the compound includes several acyl radicals, these radicals may be identical or different.

These products may be obtained, for example, by direct esterification of optionally oxyalkylenated triethanolamine, triisopropanolamine, alkyldiethanolamine or alkyldiisopropanolamine onto fatty acids or onto mixtures of fatty acids of plant or animal origin, or by transesterification of the methyl esters thereof. This esterification may be followed by a quaternization using an alkylating agent chosen from alkyl halides, for example methyl and ethyl halides; dialkyl sulfates, for example dimethyl and diethyl sulfates; methyl methanesulfonate; methyl para-toluenesulfonate; glycol chlorohydrin; and glycerol chlorohydrin.

Such compounds are sold, for example, under the names Dehyquart^{®} by the company Cognis, Stepanquat^{®} by the company Stepan, Noxamium^{®} by the company Ceca, and "Rewoquat^{®} WE 18" by the company Rewo-Goldschmidt.

Other non-limiting examples of ammonium salts that may be used in the composition according to the present invention include the ammonium salts including at least one ester function described in U.S. Pat. Nos. 4,874,554 and 4,137,180.

Among the quaternary ammonium salts mentioned above, those that may be used in the composition according to the present invention include, but are not limited to, those corresponding to formula (I), for example tetraalkylammonium chlorides, for instance dialkyldimethylammonium and alkyltrimethylammonium chlorides in which the alkyl radical includes from about 12 to 22 carbon atoms, such as behenyltrimethylammonium, distearyldimethylammonium, cetyltrimethylammonium and benzyldimethylstearylammonium chloride; palmitylamidopropyltrimethylammonium chloride; and stearamidopropyldimethyl(myristyl acetate)ammonium chloride, sold under the name "Ceraphyl^{®} 70" by the company Van Dyk.

According to one embodiment, the cationic surfactant that may be used in the composition according to the present invention is chosen from behenyltrimethylammonium chloride, cetyltrimethylammonium chloride, Quaternium-83, Quaternium-87, Quaternium-22, behenylamidopropyl-2,3-dihydroxypropyldimethylammonium chloride, palmitylamidopropyltrimethylammonium chloride, and stearamidopropyldimethylamine.

### (iv) Nonionic Surfactants

The composition comprises at least one nonionic surfactant. Two or more nonionic surfactants may be used in combination.

The nonionic surfactants are compounds well known in themselves (see, e.g., in this regard, "Handbook of Surfactants" by M. R. Porter, Blackie & Son publishers (Glasgow and London), 1991, pp. 116-178). Thus, they can, for example, be chosen from alcohols, alpha-diols, alkylphenols and esters of fatty acids, these compounds being ethoxylated, propoxylated or glycerolated and having at least one fatty chain comprising, for example, from 8 to 30 carbon atoms, it being possible for the number of ethylene oxide or propylene oxide groups to range from 2 to 50, and for the number of glycerol groups to range from 1 to 30. Maltose derivatives may also be mentioned. Non-limiting mention may also be made of copolymers of ethylene oxide and/or of propylene oxide; condensates of ethylene oxide and/or of propylene oxide with fatty alcohols; polyethoxylated fatty amides comprising, for example, from 2 to 30 mol of ethylene oxide; polyglycerolated fatty amides comprising, for example, from 1.5 to 5 glycerol groups, such as from 1.5 to 4; ethoxylated fatty acid esters of sorbitan comprising from 2 to 30 mol of ethylene oxide; ethoxylated oils of plant origin; fatty acid esters of sucrose; fatty acid esters of polyethylene glycol; polyethoxylated fatty acid mono or diesters of glycerol (C₆-C₂₄)alkylpolyglycosides; N-(C₆-C₂₄)alkylglucamine derivatives; amine oxides such as (C₁₀-C₁₄)alkylamine oxides or N-(C₁₀-C₁₄)acylaminopropylmorpholine oxides; silicone surfactants; and mixtures thereof.

The nonionic surfactants may preferably be chosen from monooxyalkylenated, polyoxyalkylenated, monoglycerolated or polyglycerolated nonionic surfactants. The oxyalkylene units are more particularly oxyethylene or oxypropylene units, or a combination thereof, and are preferably oxyethylene units.

Examples of monooxyalkylenated or polyoxyalkylenated nonionic surfactants that may be mentioned include:
monooxyalkylenated or polyoxyalkylenated (C₈-C₂₄)alkylphenols,
saturated or unsaturated, linear or branched, monooxyalkylenated or polyoxyalkylenated C₈-C₃₀ alcohols,
saturated or unsaturated, linear or branched, monooxyalkylenated or polyoxyalkylenated C₈-C₃₀ amides,
esters of saturated or unsaturated, linear or branched, C₈-C₃₀ acids and of polyalkylene glycols,
monooxyalkylenated or polyoxyalkylenated esters of saturated or unsaturated, linear or branched, C₈-C₃₀ acids and of sorbitol,
saturated or unsaturated, monooxyalkylenated or polyoxyalkylenated plant oils,
condensates of ethylene oxide and/or of propylene oxide, inter alia, alone or as mixtures.

The surfactants preferably contain a number of moles of ethylene oxide and/or of propylene oxide of between 1 and 100 and most preferably between 2 and 50. According to one of the embodiments of the present invention, the polyoxyalkylenated nonionic surfactants are chosen from polyoxyethylenated fatty alcohols (polyethylene glycol ether of fatty alcohol) and polyoxyethylenated fatty esters (polyethylene glycol ester of fatty acid).

Examples of polyoxyethylenated saturated fatty alcohols (or C₈-C₃₀ alcohols) that may be mentioned include the adducts of ethylene oxide with lauryl alcohol, especially those containing from 2 to 50 oxyethylene units and more particularly those containing from 10 to 12 oxyethylene units (Laureth-10 to Laureth-12, as the CTFA names); the adducts of ethylene oxide with behenyl alcohol, especially those containing from 2 to 50 oxyethylene units (Beheneth-9 to Beheneth-50, as the CTFA names); the adducts of ethylene oxide with cetearyl alcohol (mixture of cetyl alcohol and stearyl alcohol), especially those containing from 2 to 30 oxyethylene units (Ceteareth-10 to Ceteareth-30, as the CTFA names); the adducts of ethylene oxide with cetyl alcohol, especially those containing from 2 to 30 oxyethylene units (Ceteth-10 to Ceteth-30, as the CTFA names); the adducts of ethylene oxide with stearyl alcohol, especially those containing from 2 to 30 oxyethylene units (Steareth-2 to Steareth-30, as the CTFA names); the adducts of ethylene oxide with isostearyl alcohol, especially those containing from 2 to 50 oxyethylene units (Isosteareth-2 to Isosteareth-50, as the CTFA names); and mixtures thereof.

Examples of polyoxyethylenated unsaturated fatty alcohol (or C₈-C₃₀ alcohols) that may be mentioned include the adducts of ethylene oxide with oleyl alcohol, especially those containing from 2 to 50 oxyethylene units and more particularly those containing from 10 to 40 oxyethylene units (Oleth-10 to Oleth-40, as the CTFA names); and mixtures thereof.

As examples of monoglycerolated or polyglycerolated nonionic surfactants, monoglycerolated or polyglycerolated C₈-C₄₀ alcohols are preferably used.

In particular, the monoglycerolated or polyglycerolated C₈-C₄₀ alcohols correspond to the following formula:

RO-[CH₂-CH(CH₂OH)-O]ₘ-H or RO-[CH(CH₂OH)-CH₂O]ₘ-H

in which R represents a linear or branched C₈-C₄₀ and preferably C₈-C₃₀ alkyl or alkenyl radical, and m represents a number ranging from 1 to 30 and preferably from 1.5 to 10.

As examples of compounds that are suitable in the context of the present invention, mention may be made of lauryl alcohol containing 4 mol of glycerol (INCI name: Polyglyceryl-4 Lauryl Ether), lauryl alcohol containing 1.5 mol of glycerol, oleyl alcohol containing 4 mol of glycerol (INCI name: Polyglyceryl-4 Oleyl Ether), oleyl alcohol containing 2 mol of glycerol (INCI name: Polyglyceryl-2 Oleyl Ether), cetearyl alcohol containing 2 mol of glycerol, cetearyl alcohol containing 6 mol of glycerol, oleocetyl alcohol containing 6 mol of glycerol, and octadecanol containing 6 mol of glycerol.

The alcohol may represent a mixture of alcohols in the same way that the value of m represents a statistical value, which means that, in a commercial product, several species of polyglycerolated fatty alcohol may coexist in the form of a mixture.

Among the monoglycerolated or polyglycerolated alcohols, it is preferable to use the C₈/C₁₀ alcohol containing 1 mol of glycerol, the C₁₀/C₁₂ alcohol containing 1 mol of glycerol and the C₁₂ alcohol containing 1.5 mol of glycerol.

The monoglycerolated or polyglycerolated C₈-C₄₀ fatty esters may correspond to the following formula:

R'O-[CH₂-CH(CH₂OR‴)-O]ₘ-R" or R'O-[CH(CH₂OR‴)-CH₂O]ₘ-R"

in which each of R', R" and R‴ independently represents a hydrogen atom, or a linear or branched C₈-C₄₀ and preferably C₈-C₃₀ alkyl-CO- or alkenyl-CO-radical, with the proviso that at least one of R', R" and R‴ is not a hydrogen atom, and m represents a number ranging from 1 to 30 and preferably from 1.5 to 10.

Examples of polyoxyethylenated fatty esters that may be mentioned include the adducts of ethylene oxide with esters of lauric acid, palmitic acid, stearic acid or behenic acid, and mixtures thereof, especially those containing from 9 to 100 oxyethylene units, such as PEG-9 to PEG-50 laurate (CTFA names: PEG-9 laurate to PEG-50 laurate); PEG-9 to PEG-50 palmitate (CTFA names: PEG-9 palmitate to PEG-50 palmitate); PEG-9 to PEG-50 stearate (CTFA names: PEG-9 stearate to PEG-50 stearate); PEG-9 to PEG-50 palmitostearate; PEG-9 to PEG-50 behenate (CTFA names: PEG-9 behenate to PEG-50 behenate); polyethylene glycol 100 EO monostearate (CTFA name: PEG-100 stearate); and mixtures thereof.

According to one of the embodiments of the present invention, the nonionic surfactant may be selected from esters of polyols with fatty acids with a saturated or unsaturated chain containing for example from 8 to 24 carbon atoms, preferably 12 to 22 carbon atoms, and polyoxyalkylenated derivatives thereof, preferably containing from 10 to 200, and more preferably from 10 to 100 oxyalkylene units, such as glyceryl esters of a C₈-C₂₄, preferably C₁₂-C₂₂, fatty acid or acids and polyoxyalkylenated derivatives thereof, preferably containing from 10 to 200, and more preferably from 10 to 100 oxyalkylene units; sorbitol esters of a C₈-C₂₄, preferably C₁₂-C₂₂, fatty acid or acids and polyoxyalkylenated derivatives thereof, preferably containing from 10 to 200, and more preferably from 10 to 100 oxyalkylene units; sugar (sucrose, maltose, glucose, fructose, and/or alkylglycose) esters of a C₈-C₂₄, preferably C₁₂-C₂₂, fatty acid or acids and polyoxyalkylenated derivatives thereof, preferably containing from 10 to 200, and more preferably from 10 to 100 oxyalkylene units; ethers of fatty alcohols; ethers of sugar and a C₈-C₂₄, preferably C₁₂-C₂₂, fatty alcohol or alcohols; and mixtures thereof.

As glyceryl esters of fatty acids, mention may be made of glyceryl stearate (glyceryl mono-, di- and/or tristearate) (CTFA name: glyceryl stearate), glyceryl laurate or glyceryl ricinoleate and mixtures thereof, and as polyoxyalkylenated derivatives thereof, mention may be made of mono-, di- or triester of fatty acids with a polyoxyalkylenated glycerol (mono-, di- or triester of fatty acids with a polyalkylene glycol ether of glycerol), preferably polyoxyethylenated glyceryl stearate (mono-, di- and/or tristearate), such as PEG-20 glyceryl stearate (mono-, di- and/or tristearate).

Mixtures of these surfactants, such as for example the product containing glyceryl stearate and PEG-100 stearate, marketed under the name ARLACEL 165 by Uniqema, and the product containing glyceryl stearate (glyceryl mono- and distearate) and potassium stearate marketed under the name TEGIN by Goldschmidt (CTFA name: glyceryl stearate SE), can also be used.

The sorbitol esters of C₈-C₂₄ fatty acids and polyoxyalkylenated derivatives thereof can be selected from sorbitan palmitate, sorbitan isostearate, sorbitan trioleate and esters of fatty acids and alkoxylated sorbitan containing for example from 20 to 100 EO, such as for example sorbitan monostearate (CTFA name: sorbitan stearate), sold by the company ICI under the name Span 60, sorbitan monopalmitate (CTFA name: sorbitan palmitate), sold by the company ICI under the name Span 40, and sorbitan tristearate 20 EO (CTFA name: polysorbate 65), sold by the company ICI under the name Tween 65, polyethylene sorbitan trioleate (polysorbate 85) or the compounds marketed under the trade names Tween 20 or Tween 60 by Uniqema.

As esters of fatty acids and glucose or alkylglucose, mention may be made of glucose palmitate, alkylglucose sesquistearates such as methylglucose sesquistearate, alkylglucose palmitates such as methylglucose or ethylglucose palmitate, methylglucoside fatty esters, the diester of methylglucoside and oleic acid (CTFA name: Methyl glucose dioleate), the mixed ester of methylglucoside and the mixture of oleic acid/hydroxystearic acid (CTFA name: Methyl glucose dioleate/hydroxystearate), the ester of methylglucoside and isostearic acid (CTFA name: Methyl glucose isostearate), the ester of methylglucoside and lauric acid (CTFA name: Methyl glucose laurate), the mixture of monoester and diester of methylglucoside and isostearic acid (CTFA name: Methyl glucose sesqui-isostearate), the mixture of monoester and diester of methylglucoside and stearic acid (CTFA name: Methyl glucose sesquistearate) and in particular the product marketed under the name Glucate SS by AMERCHOL, and mixtures thereof.

As ethoxylated ethers of fatty acids and glucose or alkylglucose, mention may be made, for example, of ethoxylated ethers of fatty acids and methylglucose, and in particular the polyethylene glycol ether of the diester of methylglucose and stearic acid with about 20 moles of ethylene oxide (CTFA name: PEG-20 methyl glucose distearate) such as the product marketed under the name Glucam E-20 distearate by AMERCHOL, the polyethylene glycol ether of the mixture of monoester and diester of methyl-glucose and stearic acid with about 20 moles of ethylene oxide (CTFA name: PEG-20 methyl glucose sesquistearate) and in particular the product marketed under the name Glucamate SSE-20 by AMERCHOL and that marketed under the name Grillocose PSE-20 by GOLDSCHMIDT, and mixtures thereof.

As sucrose esters, mention may be made, for example, of saccharose palmito-stearate, saccharose stearate and saccharose monolaurate.

As sugar ethers, alkylpolyglucosides can be used, and mention may particularly be made, for example, of decylglucoside such as the product marketed under the name MYDOL 10 by Kao Chemicals, the product marketed under the name PLANTAREN 2000 by Henkel, and the product marketed under the name ORAMIX NS 10 by Seppic, caprylyl/capryl glucoside such as the product marketed under the name ORAMIX CG 110 by Seppic or under the name LUTENSOL GD 70 by BASF, laurylglucoside such as the products marketed under the names PLANTAREN 1200 N and PLANTACARE 1200 by Henkel, coco-glucoside such as the product marketed under the name PLANTACARE 818/UP by Henkel, cetostearyl glucoside possibly mixed with cetostearyl alcohol, marketed for example under the name MONTANOV 68 by Seppic, under the name TEGO-CARE CG90 by Goldschmidt and under the name EMULGADE KE3302 by Henkel, arachidyl glucoside, for example in the form of the mixture of arachidyl and behenyl alcohols and arachidyl glucoside marketed under the name MONTANOV 202 by Seppic, cocoylethylglucoside, for example in the form of the mixture (35/65) with cetyl and stearyl alcohols, marketed under the name MONTANOV 82 by Seppic, and mixtures thereof.

Mixtures of glycerides of alkoxylated plant oils such as mixtures of ethoxylated (200 EO) palm and copra (7 EO) glycerides can also be cited.

The nonionic surfactant according to the present invention preferably contains alkenyl or a branched C₁₂-C₂₂ acyl chain such as an oleyl or isostearyl group. More preferably, the nonionic surfactant according to the present invention is PEG-20 glyceryl triisostearate.

According to one of the embodiments of the present invention, the nonionic surfactant may be selected from copolymers of ethylene oxide and of propylene oxide, in particular copolymers of the following formula:

HO(C₂H₄O)ₐ(C₃H₆O)_{b}(C₂H₄O)_{c}H

in which a, b and c are integers such that a+c ranges from 2 to 100 and b ranges from 14 to 60, and mixtures thereof.

According to one of the embodiments of the present invention, the nonionic surfactant may be selected from silicone surfactants. Non-limiting mention may be made of those disclosed in documents US-A-5364633 and US-A-5411744.

The silicone surfactant may preferably be a compound of formula (I): 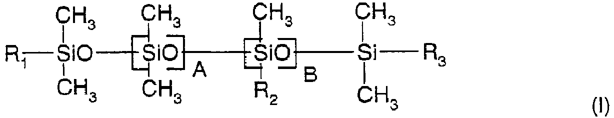 in which:
R₁, R₂ and R₃, independently of each other, represent a C₁-C₆ alkyl radical or a radical -(CH₂)ₓ-(OCH₂CH₂)_{y}-(OCH₂CH₂CH₂)_{z}-OR₄, at least one radical R₁, R₂ or R₃ not being an alkyl radical; R₄ being a hydrogen, an alkyl radical or an acyl radical;
A is an integer ranging from 0 to 200;
B is an integer ranging from 0 to 50; with the proviso that A and B are not simultaneously equal to zero;
x is an integer ranging from 1 to 6;
y is an integer ranging from 1 to 30;
z is an integer ranging from 0 to 5.

According to one preferred embodiment of the present invention, in the compound of formula (I), the alkyl radical is a methyl radical, x is an integer ranging from 2 to 6 and y is an integer ranging from 4 to 30.

As examples of silicone surfactants of formula (I), mention may be made of the compounds of formula (II): 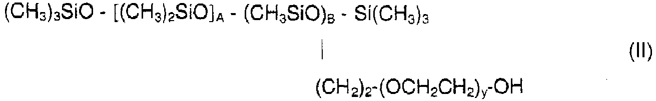 in which A is an integer ranging from 20 to 105, B is an integer ranging from 2 to 10 and y is an integer ranging from 10 to 20.

As examples of silicone surfactants of formula (I), mention may also be made of the compounds of formula (III):

H-(OCH₂CH₂)_{y}-(CH₂)₃-[(CH₃)₂SiO]_{A'}-(CH₂)₃-(OCH₂CH₂)_{y}-OH (III)

in which A' and y are integers ranging from 10 to 20.

Compounds of the present invention which may be used are those sold by the company Dow Corning under the names DC 5329, DC 7439-146, DC 2-5695 and Q4-3667. The compounds DC 5329, DC 7439-146 and DC 2-5695 are compounds of formula (II) in which, respectively, A is 22, B is 2 and y is 12; A is 103, B is 10 and y is 12; and A is 27, B is 3 and y is 12.

The compound Q4-3667 is a compound of formula (III) in which A is 15 and y is 13.

### (Conditioning Agent)

The composition for straightening keratin fibers used in the process according to the present invention may further comprise at least one conditioning agent. Two or more conditioning agents may be used in combination. Thus, a single type of conditioning agent or a combination of different types of conditioning agents may be used.

The conditioning agent can provide keratin fibers such as hair with conditioning effects.

It is preferable that the conditioning agent be selected from cationic polymers.

The composition according to the present invention may comprise at least one cationic polymer. A single type of cationic polymer may be used, but two or more different types of cationic polymers may be used in combination.

It should be noted that, for the purposes of the present invention, the term "cationic polymer" denotes any polymer containing cationic groups and/or groups that may be ionized into cationic groups.

Such polymers may be chosen from those already known per se as improving the cosmetic properties of the hair, i.e., especially those described in patent application EP-A-337 354 and in French patents FR-2 270 846, 2 383 660, 2 598 611, 2 470 596 and 2 519 863.

The cationic polymers that are preferred are chosen from those containing units comprising primary, secondary, tertiary and/or quaternary amine groups, which may either form part of the main polymer chain or may be borne by a side substituent directly attached thereto.

The cationic polymers used generally have a number-average molecular mass of between approximately 500 and approximately 5×10⁶ and preferably between approximately 10³ and approximately 3×10⁶.

Among the cationic polymers that may be mentioned more particularly are polymers of the polyamine, polyamino amide and polyquaternary ammonium type.

These are known products. They are described in particular in French patents 2 505 348 and 2 542 997. Among the said polymers, mention may be made of the following.
(1) homopolymers or copolymers derived from acrylic or methacrylic esters or amides and comprising at least one of the units of formula (I), (II), (III) or (IV) below: 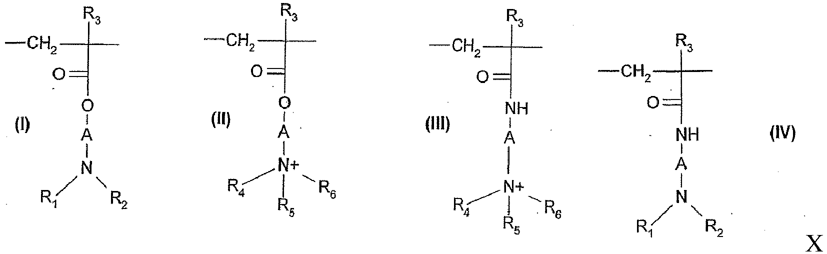 in which
   R₃, which may be identical or different, denote a hydrogen atom or a CH₃ radical;
   A, which may be identical or different, represent a linear or branched alkyl group of 1 to 6 carbon atoms, preferably 2 or 3 carbon atoms, or a hydroxyalkyl group of 1 to 4 carbon atoms;
   R₄, R₅ and R₆, which may be identical or different, represent an alkyl group containing from 1 to 18 carbon atoms or a benzyl radical and preferably an alkyl group containing from 1 to 6 carbon atoms;
   R₁ and R₂, which may be identical or different, represent hydrogen or an alkyl group containing from 1 to 6 carbon atoms, and preferably methyl or ethyl; and
   X denotes an anion derived from an inorganic or organic acid, such as a methosulfate anion or a halide such as chloride or bromide.

The polymers of family (1) can also contain one or more units derived from comonomers which may be chosen from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinyl-caprolactam, and vinyl esters.

Thus, among these polymers of family (1), mention may be made of:
- copolymers of acrylamide and of dimethylaminoethyl methacrylate quaternized with dimethyl sulfate or with a dimethyl halide, such as the product sold under the name Hercofloc by the company Hercules,
- the copolymers of acrylamide and of methacryloyloxyethyltrimethylammonium chloride described, for example, in patent application EP-A-080 976 and sold under the name Bina Quat P 100 by the company BASF,
- the copolymer of acrylamide and of methacryloyloxyethyltrimethylammonium methosulfate sold under the name Reten by the company Hercules,
- quaternized or non-quaternized vinylpyrrolidone/dialkylaminoalkyl acrylate or methacrylate copolymers, such as the products sold under the name "Gafquat" by the company ISP, for instance "Gafquat 734" or "Gafquat 755", or alternatively the products known as "Copolymer 845, 958 and 937". These polymers are described in detail in French patents 2077 143 and 2 393 573,
- dimethylaminoethyl methacrylate/vinylcaprolactam/vinylpyrrolidone terpolymers, such as the product sold under the name Gaffix VC 713 by the company ISP, and
- vinylpyrrolidone/methacrylamidopropyldimethylamine copolymers sold in particular under the name Styleze CC 10 by ISP, and quaternized vinylpyrrolidone/dimethylaminopropyl methacrylamide copolymers such as the product sold under the name "Gafquat HS 100" by the company ISP.

(2) The cellulose ether derivatives comprising quaternary ammonium groups, which are described in French patent 1 492 597, and in particular the polymers sold under the names "JR" (JR 400, JR 125, JR 30M) or "LR" (LR 400, LR 30M) by the company Amerchol. These polymers are also defined in the CTFA dictionary as hydroxyethylcellulose quaternary ammoniums that have reacted with an epoxide substituted with a trimethylammonium group.

(3) Cationic cellulose derivatives such as the copolymers of cellulose or cellulose derivatives grafted with a water-soluble quaternary ammonium monomer, described especially in US patent 4 131 576, such as hydroxyalkylcelluloses, for instance hydroxymethyl-, hydroxyethyl- or hydroxypropylcelluloses grafted especially with a methacryloylethyl-trimethylammonium, methacrylamidopropyltrimethylammonium or dimethyldiallylammonium salt.

The commercial products corresponding to this definition are more particularly the products sold under the name Celquat L 200 and Celquat H 100 by the company Akzo Nobel.

(4) The cationic guar gums described more particularly in US patents 3 589 578 and 4 031 307, such as guar gums containing trialkylammonium cationic groups. Use is made, for example, of guar gums modified with a salt (e.g., chloride) of 2,3-epoxypropyltrimethylammonium. Mention may be made of guar hydroxypropyltrimonium chloride and hydroxypropyl guar hydroxypropyl trimonium chloride, such as those sold especially under the trade names Jaguar C13S, Jaguar C14S, Jaguar C17 and Jaguar C162 by the company Solvay.

(5) Polymers consisting of piperazinyl units and of divalent alkylene or hydroxyalkylene radicals containing straight or branched chains, optionally interrupted by oxygen, sulfur or nitrogen atoms or by aromatic or heterocyclic rings, and also the oxidation and/or quaternization products of these polymers. Such polymers are described, in particular, in French patents 2 162 025 and 2 280 361.

(6) Water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized. Such polymers are described, in particular, in French patents 2 252 840 and 2 368 508.

(7) Cyclopolymers of alkyldiallylamine or of dialkyldiallylammonium, such as the homopolymers or copolymers containing, as a main constituent of the chain, units corresponding to formula (V) or (VI): 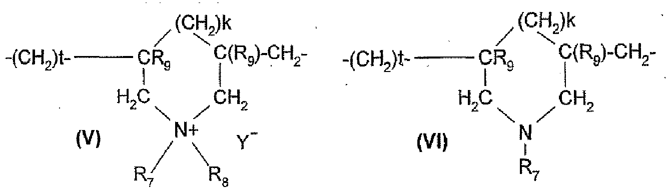 in which formulae
k and t are equal to 0 or 1, the sum k + t being equal to 1; R₉ denotes a hydrogen atom or a methyl radical; R₇ and R₈, independently of each other, denote an alkyl group having from 1 to 6 carbon atoms, a hydroxyalkyl group in which the alkyl group preferably has 1 to 5 carbon atoms, a lower (C₁-C₄) amidoalkyl group, or R₇ and R₈ can denote, together with the nitrogen atom to which they are attached, heterocyclic groups such as piperidyl or morpholinyl; R₇ and R₈, independently of each other, preferably denote an alkyl group having from 1 to 4 carbon atoms; and Y⁻ is an anion such as bromide, chloride, acetate, borate, citrate, tartrate, bisulfate, bisulfite, sulfate or phosphate. These polymers are described in particular in French patent 2 080 759 and in its Certificate of Addition 2 190 406.

Among the polymers defined above, mention may be made more particularly of the dimethyldiallylammonium chloride homopolymer sold under the name "Merquat 100" by the company Nalco (and its homologues of low weight-average molecular mass) and the copolymers of diallyldimethylammonium chloride and of acrylamide, sold under the name "Merquat 550".

(8) The quaternary diammonium polymer containing repeating units corresponding to the formula: 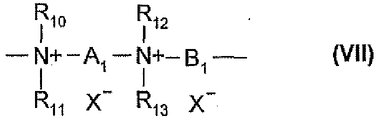 in which formula (VII):
R₁₀, R₁₁, R₁₂ and R₁₃, which may be identical or different, represent aliphatic, alicyclic or arylaliphatic radicals containing from 1 to 20 carbon atoms or lower hydroxyalkylaliphatic radicals, or alternatively R₁₀, R₁₁, R₁₂ and R₁₃, together or separately, constitute, with the nitrogen atoms to which they are attached, heterocycles optionally containing a second hetero atom other than nitrogen, or alternatively R₁₀, R₁₁, R₁₂ and R₁₃ represent a linear or branched C₁-C₆ alkyl radical substituted with a nitrile, ester, acyl or amide group or a group -CO-O-R₁₄-D or -CO-NH-R₁₄-D where R₁₄ is an alkylene and D is a quaternary ammonium group;
A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
X⁻ denotes an anion derived from an inorganic or organic acid;
A₁, R₁₀ and R₁₂ can form, with the two nitrogen atoms to which they are attached, a piperazine ring; in addition, if A₁ denotes a linear or branched, saturated or unsaturated alkylene or hydroxyalkylene radical, B₁ can also denote a group -(CH₂)ₙ-CO-D-OC-(CH₂)ₙ- in which D denotes:
   i) a glycol residue of formula: -O-Z-O-, where Z denotes a linear or branched hydrocarbon-based radical or a group corresponding to one of the following formulae:

      -(CH₂-CH₂-O)ₓ-CH₂-CH₂-;

      and

      -[CH₂-CH(CH₃)-O]_{y}-CH₂-CH(CH₃)-

      where x and y denote an integer from 1 to 4, representing a defined and unique degree of polymerization or any number from 1 to 4 representing an average degree of polymerization;
   ii) a bis-secondary diamine residue such as a piperazine derivative;
   iii) a bis-primary diamine residue of formula -NH-Y-NH-, where Y denotes a linear or branched hydrocarbon-based radical, or alternatively the divalent radical

      -CH₂-CH₂-S-S-CH₂-CH₂-;

      or
   iv) a ureylene group of formula -NH-CO-NH-.

Preferably, X⁻ is an anion such as chloride or bromide.

These polymers generally have a number-average molecular mass of between 1000 and 100000.

Polymers of this type are described in particular in French patents 2 320 330, 2 270 846, 2 316 271, 2 336 434 and 2 413 907 and US patents 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 and 4 027 020.

It is more particularly possible to use polymers that consist of repeating units corresponding to the following formula (VIII): 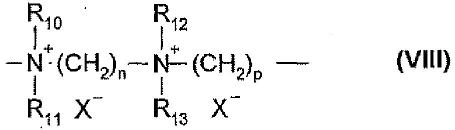 in which
R₁₀, R₁₁, R₁₂ and R₁₃, which may be identical or different, denote an alkyl or hydroxyalkyl radical containing from 1 to 4 carbon atoms approximately, n and p are integers ranging from 2 to 20 approximately, and X⁻ is an anion derived from a mineral or organic acid.

One particularly preferred compound of formula (VIII) is that for which R₁₀, R₁₁ R₁₂ and R₁₃ represent a methyl group, n=3, p=6 and X=Cl, which is called hexadimethrine chloride according to the INCI(CTFA) nomenclature.

(9) Polyamines such as Polyquart H sold by Cognis, which is given under the reference name "Polyethylene glycol (15) tallow polyamine" in the CTFA dictionary.

(10) Crosslinked methacryloyloxy(C₁-C₄)alkyltri(C₁-C₄)alkylammonium salt polymers such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound containing olefinic unsaturation, in particular methylenebisacrylamide. A crosslinked acrylamide/methacryloyloxyethyltrimethylammonium chloride copolymer (20/80 by weight) in the form of a dispersion containing 50% by weight of the said copolymer in mineral oil can be used more particularly. This dispersion is sold under the name "Salcare^{®} SC 92" by the company BASF. A crosslinked methacryloyloxyethyltrimethylammonium chloride homopolymer containing about 50% by weight of the homopolymer in mineral oil or in a liquid ester can also be used. These dispersions are sold under the names "Salcare^{®} SC 95" and "Salcare^{®} SC 96" by the company Allied Colloids.

(11) Other cationic polymers which can be used in the context of the present invention are polyalkyleneimines, in particular polyethyleneimines, polymers containing vinylpyridine or vinylpyridinium units, condensates of polyamines and of epichlorohydrin, quaternary polyureylenes and chitin derivatives.

It is preferable that the cationic polymer be a polyquaternium polymer or a polymeric quaternary ammonium salt.

Polymeric quaternary ammonium salts are cationic polymers comprising at least one quaternized nitrogen atom. Mention may in particular be made, as polymeric quaternary ammonium salts, of the Polyquaternium products (CTFA name), which contribute mainly to the quality of foam and feeling of the skin after use, in particular the feeling of the skin after use. These polymers can preferably be chosen from the following polymers:
Polyquaternium-5, such as the product Merquat 5 sold by Nalco;
Polyquaternium-6, such as the product Salcare SC 30 sold by BASF and the product Merquat 100 sold by Nalco;
Polyquaternium-7, such as the products Merquat S, Merquat 2200, Merquat 7SPR, and Merquat 550 sold by Nalco and the product Salcare SC 10 sold by BASF;
Polyquaternium-10, such as the product Polymer JR400 sold by Amerchol;
Polyquaternium-11, such as the products Gafquat 755, Gafquat 755N and Gafquat 734 sold by ISP;
Polyquaternium-15, such as the product Rohagit KF 720 F sold by Röhm;
Polyquaternium-16, such as the products Luviquat FC905, Luviquat FC370, Luviquat HM552 and Luviquat FC550 sold by BASF;
Polyquaternium-28, such as the product Styleze CC10 sold by ISP;
Polyquaternium-44, such as the product Luviquat Care sold by BASF;
Polyquaternium-46, such as the product Luviquat Hold sold by BASF;
Polyquaternium-47, such as the product Merquat 2001 sold by Nalco; and
Polyquaternium-67, such as the product Softcat SL-5, SL-30, SL-60 and SL-100 sold by Amerchol.

Preferably, the cationic polymer is chosen from, Polyquaternium-67, hexadimethrine chloride and their mixtures.

The amount of the conditioning agent(s) in the composition for the process according to the present invention may be 0.01% by weight or more, preferably 0.05% by weight or more, and more preferably 0.1% by weight or more, relative to the total weight of the composition.

On the other hand, the amount of the conditioning agent(s) in the composition for the process according to the present invention may be 10% by weight or less, preferably 5% by weight or less, and more preferably 1% by weight or less, relative to the total weight of the composition.

The amount of the conditioning agent(s) in the composition for the process according to the present invention may be from 0.01 to 10% by weight, preferably 0.05 to 5% by weight, and more preferably 0.1 to 1% by weight, relative to the total weight of the composition.

### (Monovalent Alcohol)

The composition for straightening keratin fibers used in the process according to the present invention may further comprise at least one monovalent alcohol. Two or more such alcohols may be used in combination. Thus, a single type of such alcohol or a combination of different types of such alcohol may be used.

The monovalent alcohol is preferably in the form of a liquid at ambient temperature such as 25°C under atmospheric pressure (760 mmHg or 10⁵Pa).

The term "monovalent alcohol" here means an alcohol having one hydroxy group.

The monovalent alcohol may be non-aromatic (aliphatic) or aromatic.

The non-aromatic monovalent alcohol is preferably a saturated or unsaturated, linear or branched lower aliphatic monovalent alcohol, more preferably a C₂-C₆ aliphatic monovalent alcohol, even more preferably a saturated or unsaturated, linear or branched C₂-C₅ aliphatic monovalent alcohol, and most preferably a saturated or unsaturated, linear or branched C₂-C₄ aliphatic monovalent alcohol. Preferred non-aromatic monovalent alcohols are ethanol, isopropanol and mixtures thereof.

The aromatic monovalent alcohol is preferably selected from the group consisting of benzyl alcohol, phenethylalcohol, diphenyl ethanol, cinnamyl alcohol, tryptophol, 3-nitrobenzylalcohol, veratryl alcohol, benzoin and mixtures thereof.

It is preferable that the monovalent alcohol not be a fatty alcohol or higher alcohol.

It is preferable that the monovalent alcohol be selected from the group consisting of lower aliphatic alcohols, aromatic alcohols and mixtures thereof, and more preferably selected from the group consisting of ethanol, benzyl alcohol, and mixtures thereof.

The amount of the monovalent alcohol(s) in the composition for the process according to the present invention may be 0.01% by weight or more, preferably 0.1% by weight or more, and more preferably 1% by weight or more, relative to the total weight of the composition.

On the other hand, the amount of the monovalent alcohol(s) in the composition for the process according to the present invention may be 20% by weight or less, preferably 15% by weight or less, and more preferably 10% by weight or less, relative to the total weight of the composition.

The amount of the monovalent alcohol(s) in the composition according to the present invention may range from 0.01% to 20% by weight, preferably from 0.1% to 15% by weight, and more preferably from 1% to 10% by weight, relative to the total weight of the composition.

### (Thickener)

The composition for straightening keratin fibers used in the process according to the present invention may comprise at least one thickener. A single type of thickener may be used, but two or more different types of thickener may be used in combination.

The thickener may be selected from hydrophilic polymers, preferably water-soluble polymers.

It is preferable that the thickener be selected from the group consisting of:
(i) associative thickeners;
(ii) crosslinked acrylic acid homopolymers;
(iii) crosslinked copolymers of (meth)acrylic acid and of (C₁-C₆)alkyl acrylate;
(iv) nonionic homopolymers and copolymers comprising ethylenically unsaturated monomers of ester and/or amide type;
(v) ammonium acrylate homopolymers and copolymers of ammonium acrylate and of acrylamide;
(vi) polysaccharides; and
(vii) C₁₂-C₃₀ fatty alcohols.

It is preferable that the thickener is selected from polysaccharides.

The polysaccharides are, for example, chosen from glucans, modified and unmodified starches (such as those derived, for example, from cereals, for instance wheat, corn, or rice, from vegetables, for instance yellow peas, and tubers, for instance potatoes or cassava), amylose, amylopectin, glycogen, dextrans, celluloses, and derivatives thereof (e.g., methylcelluloses, hydroxyalkylcelluloses, ethyl hydroxyethylcelluloses, and carboxymethylcelluloses), mannans, xylans, lignins, arabans, galactans, galacturonans, chitin, chitosans, glucuronoxylans, arabinoxylans, xyloglucans, glucomannans, pectic acids, and pectins, alginic acid and alginates, arabinogalactans, carrageenans, agars, glycosaminoglucans, gum arabics, gum tragacanths, ghatti gums, karaya gums, carob gums, galactomannans, such as guar gums, and nonionic derivatives thereof (e.g., hydroxypropyl guar), and xanthan gums, and mixtures thereof.

The amount of the thickener(s) in the composition for the process according to the present invention may be 0.01% by weight or more, preferably 0.05% by weight or more, and more preferably 0.1% by weight or more, relative to the total weight of the composition.

On the other hand, the amount of the thickener(s) in the composition for the process according to the present invention may be 10% by weight or less, preferably 5% by weight or less, and more preferably 3% by weight or less, relative to the total weight of the composition.

The amount of the thickener(s) in the composition for the process according to the present invention may be from 0.01% to 10% by weight, preferably 0.05% to 5% by weight, and more preferably 0.1% to 3% by weight, relative to the total weight of the composition.

### (Other Ingredients)

The composition for straightening keratin fibers used in the process according to the present invention may be aqueous or anhydrous. It is preferable that the composition comprise water at a concentration ranging from 10% to 90%, preferably from 30% to 80% and more preferably from 50% to 70% by weight relative to the total weight of the composition.

The composition for straightening keratin fibers used in the process according to the present invention may also comprise at least one additional ingredient.

The amount of the additional ingredient(s) is not limited, but may be from 0.01% to 10% by weight relative to the total weight of the composition.

The additional ingredient(s) may be selected from the group consisting of hydrophilic thickeners such as hydroxypropyl guar; hydrophobic thickeners; anionic, nonionic or amphoteric polymers; peptides and derivatives thereof; protein hydrolyzates; swelling agents and penetrating agents; agents for combating hair loss; anti-dandruff agents; suspending agents; sequestering agents; opacifying agents; dyes; sunscreen agents; vitamins or provitamins; fragrances; preserving agents, stabilizers; and mixtures thereof.

The compositions used for the process according to the invention may be in any of the formulation forms conventionally used, and in particular in the form of an aqueous, alcoholic or aqueous-alcoholic, or oily solution or suspension; a solution or a dispersion of the lotion or serum type; an emulsion, in particular of liquid or semi-liquid consistency, of the O/W, W/O or multiple type; a suspension or emulsion of soft consistency of cream (O/W) or (W/O) type; an aqueous or anhydrous gel, or any other cosmetic form.

### (pH)

The composition used for the process according to the present invention has a pH of from 7.5 to 12.0, preferably from 8.0 to 11.5, and more preferably from 8.5 to 11.0, which is measured at 25°C.

Thus, the composition used for the process according to the present invention is not anhydrous.

It is preferable that the pH of the composition used for the process according to the present invention be within ±2 relative to a pH which is equal to the pKa of the following equilibrium:

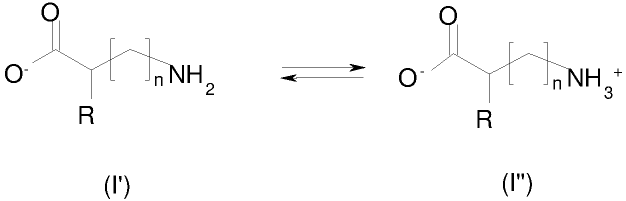

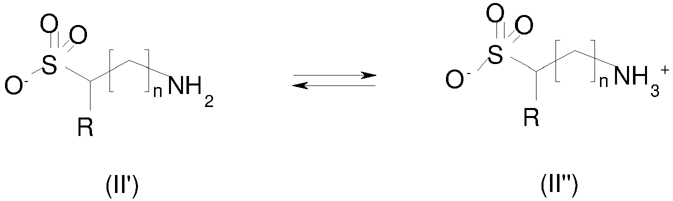

### (Ammonia and Thiol Compound)

It is preferable that the composition used for the process according to the present invention be free of ammonia or a thiol compound. The term "free of ammonia or a thiol compound" means that the composition used for the process according to the present invention does not include a substantial amount of ammonia or a thiol compound. Preferably the composition used in the process according to the present invention includes 1% by weight or less, more preferably 0.5% by weight or less, and even more preferably 0.1% by weight or less of ammonia or a thiol compound, in particular no ammonia or the thiol compound.

Due to the very small amount or the absence of ammonia and/or thiol compound, malodor during the use of the composition used for the process according to the present invention can be reduced or prevented.

The thiol compound here means a compound which has at least one thiol (-SH) group.

The thiol compound may be a reducing agent. The thiol reducing agent may be chosen from the group consisting of thioglycolic acid and derivatives thereof, in particular esters thereof such as glycerol or glycol monothioglycolate; thiolactic acid and derivatives thereof, in particular esters thereof such as glycerol monothiolactate; 3-mercaptopropionic acid and derivatives thereof, in particular esters thereof such as glycerol 3-mercaptopropionate and ethyleneglycol 3-mercaptopropionate; cysteamine and derivatives thereof, in particular C₁-C₄ acyl derivatives thereof such as N-acetylcysteamine and N-propionylcysteamine; mono-thioglycerol and derivatives thereof, in particular esters; cysteine and derivatives thereof, in particular esters such as N-acetylcysteine, N-alkanoylcysteine and cysteine alkyl esters; thioglycerine and derivatives thereof, in particular s-alkyl derivatives, and salts thereof.

As the above salts, mention may be made of, for example, ammonium salts; primary-, secondary- or tertiary-amine salts; alkaline metal salts; and, alkaline earth metal salts. As the primary-, secondary- or tertiary-amine, mention may be made of, for example, monoethanolamine, di-isopropanolamine or triethanolamine, respectively.

Other examples of the thiol reducing agent include, but are not limited to, sugar N-mercapto alkyl amides such as N-(mercapto-2-ethyl)gluconamide, β-mercaptopropionic acid and derivatives thereof; thiomalic acid; pantheteine; N-(mercaptoalkyl)ω-hydroxyalkyl amides such as those described in European Patent Application No. 0 354 835 and N-mono- or N,N-dialkylmercapto 4-butyramides such as those described in European Patent Application No. 0 368 763; aminomercaptoalkyl amides such as those described in European Patent Application No. 0 432 000 and alkylaminomercaptoalkylamides such as those described in European Patent Application No. 0 514 282; (2/3) hydroxy-2 propyl thioglycolate; and the hydroxy-2 methyl-1 ethyl thioglycolate-based mixture (67/33) described in French Patent Application No. 2 679 448.

### (Reducing Agent and Oxidizing Agent)

The composition used for the process according to the present invention may comprise a reducing agent; however, it is preferable that the composition used for the process according to the present invention comprises a reduced amount of a reducing agent or an oxidizing agent, preferably free of a reducing agent or an oxidizing agent.

The term "free of a reducing agent or an oxidizing agent" means that the composition used for the process according to the present invention does not include a substantial amount of a reducing agent or an oxidizing agent. Preferably the composition used for the process according to the present invention includes 1% by weight or less, more preferably 0.5% by weight or less, and even more preferably 0.1% by weight or less of a reducing agent or an oxidizing agent, in particular no reducing agent or no oxidizing agent.

The reducing agent may be a thiol reducing agent or a non-thiol reducing agent. The thio reducing agent is as described above.

The non-thiol reducing agent here means a reducing agent with no thiol group. The non-thiol reducing agent may be chosen from the group consisting of sulfites, bisulfites, sulfinates, phosphines, sugars, reductones and hydrides. The non-thiol reducing agent may be selected from ammonium sulfites and bisulfites as well as metal sulfites and bisulfites, more preferably alkali metal or alkali earth metal sulfites and bisulfites, and more preferably sodium sulfites and bisulfites.

The oxidizing agent may be chosen from hydrogen peroxide, alkali metal bromates, ferricyanides peroxygenated salts, and compounds capable of producing hydrogen peroxide by hydrolysis. For example, the oxidizing agent can be chosen from aqueous hydrogen peroxide solution, urea peroxide, alkali metal bromates and persalts such as perborates and persulphates

According to one embodiment of the process according to the present invention, no or very little reducing or oxidizing agent may be used to straighten keratin fibers such as hair. Therefore, as compared to conventional straightening processes for keratin fibers which require reducing/oxidizing of the keratin fibers, the process according to the present invention can reduce the time required for straightening the keratin fibers. Furthermore, one embodiment of the process according to the present invention may use no or very little reducing or oxidizing agent, and therefore, the damage to the keratin fibers can be reduced as compared to the conventional processes which require the use of the reducing or oxidizing agent.

### EXAMPLES

The present invention will be described in a more detailed manner by way of examples.

### [Composition]

An emulsion-type composition based on the following formulation shown in Table 1 was prepared by mixing the ingredients shown in Table 1. The numerical values for the amounts of the ingredients shown in Table 1 are all based on "% by weight" as ingredients.

**Table 1**

| Ingredients | |
|---|---|
| Farnesane | 5.00 |
| Cetearyl Alcohol | 2.50 |
| Steareth-2 | 2.00 |
| Steareth-20 | 2.00 |
| Pentylene Glycol | 5.00 |
| Hydropypropyl Guar | 1.00 |
| Polyquaternium-67 | 0.50 |
| Dimethicone | 0.20 |
| Hexadimethrine Chloride | 0.30 |
| NaOH | 1.20 |
| Monoethanolamine | 1.22 |
| Taurine | 7.80 |
| Magnesium Gluconate | 0.25 |
| Ethanol | 5.00 |
| Water | qsp 100 |

### [Examples 1-2 and Comparative Examples 1-6]

The above composition was used to straighten a hair swatch, and then the hair swatch was evaluated, in accordance with the following protocols.
1) The above composition (for Examples 1-2 and Comparative Examples 2-6) or water (for Comparative Example 1) was applied onto a pre-shampooed Brazilian natural curly hair swatch (20 cm length) at a weight ratio of 1:1 (composition:hair).
2) For Comparative Examples 3-5, the hair swatch was left with the composition for 30 minutes, and then dried with a hair drier. For Examples 1-2 and Comparative Example 1-2 and 6, this step was omitted, and went to step 3 or 4 right after step 1 (applying the composition onto the hair swatch).
3) For Examples 1-2 and Comparative Examples 1, 4 and 6, the hair swatch was wrapped with aluminum foil.
4) The hair swatch (with or without wrapping) was subjected to straightening by strokes with a straight iron under different conditions (at 90 or 120°C for 1 or 3 minutes, 10 second/stroke for Comparative Examples 1-4 and 6, and Examples 1-2, or at 210°C for 1 minute, 6 second/stroke for Comparative Example 5).
5) The hair swatch was cooled to room temperature within 2-3 minutes, followed by rinsing off the composition with tap water for 1 min and then drying.
6) The straightening level of the hair swatch was evaluated visually just after step 5 (for straightening effect at T=0).
7) The hair swatch was shampooed once and dried.
8) The straightening level of the hair swatch was evaluated visually again (for lasting effect after the 1 shampoo). Also, additional evaluations were made for easy combing as well as softness and smoothness after the first shampooing.
9) Shampooing and drying the hair swatch were repeated 9 times
10) The straightening level of the hair swatch was evaluated visually again (for lasting effect after the tenth shampooing).

The criteria of the above evaluations were as follows.

### (Straightening/Lasting Effects)

| | |
|---|---|
| Good: | The hair of the hair swatch was straight |
| Fair: | Some waves remained on the hair of the hair swatch |
| Poor: | many waves remained on the hair of the hair swatch |

### (Easy Coming)

| | |
|---|---|
| Good: | Needs less than 5 combings to detangle hair |
| Fair: | Needs 5 to 10 combings to detangle hair |
| Poor: | Need more than 10 combings to detangle hair |

### (Softness and Smoothness)

| | |
|---|---|
| Good: | Softer and smoother than before the treatment |
| Fair: | Same softness and smoothness as before the treatment |
| Poor: | Less soft and less smooth than before the treatment |

The results of the above evaluations are shown in Table 2.

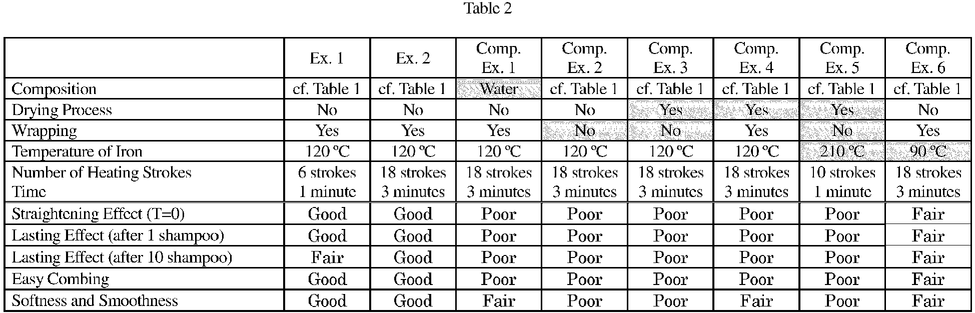

The straightening process according to Example 1 provided hair with good immediate straightening and lasting effects. It was easy to comb the hair treated with the straightening process according to Example 1. The hair treated with the straightening process according to Example 1 was soft and smooth which indicates that conditioning effects were provided to the hair.

The straightening process according to Example 2 provided hair with good immediate straightening and long lasting effects. It was easy to comb the hair treated with the straightening process according to Example 2. The hair treated with the straightening process according to Example 2 was soft and smooth which indicates that conditioning effects were provided to the hair.

The straightening process according to Comparative Example 1 using only water provided hair with poor immediate straightening and lasting effects. It was not easy to comb the hair treated with the straightening process according to Comparative Example 1.

The straightening process according to Comparative Example 2, in which the hair was not wrapped, provided hair with poor immediate straightening and lasting effects. Since heating iron was directly applied on the wet hair, water was evaporated during the heating, and undesired curl shape setting was happened due to excessive contractions. Also, it was not easy to comb the hair treated with the straightening process according to Comparative Example 2 due to the hair damage caused by direct heating. In fact, the hair treated with the straightening process according to Comparative Example 2 was not soft and not smooth, which indicate that damage was provided to the hair. In particular, based on the comparison between Example 1 and Comparative Example 2, it is clearly indicated that wrapping hair is necessary to provide the desired effects.

The straightening process according to Comparative Example 3, in which the hair was dried and not wrapped, provided hair with poor immediate straightening and lasting effects. Thus, it indicates that a wet condition during heating is necessary for hair. Also, it was not easy to comb the hair treated with the straightening process according to Comparative Example 3 due to the hair damage caused by direct heating. In fact, the hair treated with the straightening process according to Comparative Example 3 was not soft and not smooth which indicates that damage was provided to the hair.

The straightening process according to Comparative Example 4, in which the hair was dried, provided hair with poor immediate straightening and lasting effects. Thus, it indicates again that a wet condition during heating is necessary for hair. Also, it was not easy to comb the hair treated with the straightening process according to Comparative Example 4. In particular, based on the comparison between Example 1 and Comparative Example 4, it is clearly indicated that keeping hair wet during heating is necessary to provide the desired effects.

The straightening process according to Comparative Example 5, in which the hair was dried and not wrapped, provided hair with poor immediate straightening and lasting effects. Thus, it indicates that a wet condition during heating is necessary for hair. Also, it was not easy to comb the hair treated with the straightening process according to Comparative Example 5 due to the hair damage caused by direct heating at a higher temperature. In fact, the hair treated with the straightening process according to Comparative Example 5 was not soft and not smooth which indicates that damage was provided to the hair.

The straightening process according to Comparative Example 6, in which the hair was heated at 90 °C, provided hair with some immediate straightening and lasting effects. Also, it was OK to comb the hair treated with the straightening process according to Comparative Example 6. However, the straightening effects thus obtained were not sufficient. In particular, based on the comparison between Example 1 and Comparative Example 6, it is clearly indicated that heating hair over 100 °C is necessary to provide the desired effects.

### (Damage Evaluation)

The tensile strength of a hair fiber treated with the process according to Example 1 or Example 2, and that of a hair fiber treated with a process, which is the same as the process according to Example 1, were measured to compare the break stresses of the hair fibers with the proviso that the composition shown in Table 1 was replaced with a conventional straightening perm product.

The break stress of the hair fiber treated with the process according to Example 1 or Example 2 was significantly higher (stronger) than that of the hair fiber treated with the comparative process using the conventional straightening perm product.

The above results indicate that the present invention causes less damage than conventional straightening perm products.

## Claims

1. A process for straightening keratin fibers, preferably hair, comprising the steps of:
(i) applying onto the keratin fibers a composition for straightening keratin fibers;
(ii) wrapping the keratin fibers, onto which the composition is applied, with at least one wrapping means in order to keep the keratin fibers wet;
(iii) straightening the wet keratin fibers with a heating iron at a temperature of more than 100 °C, preferably more than 100 °C and less than 210 °C, and more preferably from 110 °C to 200 °C;
(iv) unwrapping the keratin fibers; and
(v) optionally rinsing and/or drying the keratin fibers,
wherein
the composition for straightening keratin fibers comprises
(a) at least one compound chosen from alkylaminosulfonic acids and compounds of the following formulae (I) and (II): 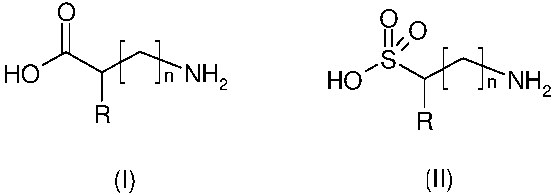 in which in formulae (I) and (II)
- R represents a hydrogen atom, or a linear or branched, preferably linear, C₁-C₅ alkyl group, said alkyl group being optionally substituted with at least one group chosen from a hydroxyl group, an amino group, a carboxamido group, a C₆-C₁₈ aromatic group, a heterocyclic group, -C(O)-OH, -S(O)₂-OH, -C(O)-O⁻M⁺, -S(O)₂-O⁻M⁺, and mixtures thereof with M⁺ representing a cationic counterion such as an alkali metal, alkaline-earth metal, or ammonium, and
- n is 0 or 1,
and
b) monoethanolamine,
and
the composition for straightening keratin fibers has a pH of from 7.5 to 12.0, preferably from 8.0 to 11.5, and more preferably from 8.5 to 11.0.

2. The process according to claim 1, wherein the wrapping means is heat-resistant, preferably resistant to a temperature of more than 100 °C, more preferably 110 °C or more, and even more preferably 120 °C or more.

3. The process according to claim 1 or 2, wherein the wrapping means forms at least one occlusive space including the keratin fibers.

4. The process according to any one of claims 1 to 3, wherein the pH of the composition for straightening keratin fibers is within ±2 relative to a pH which is equal to the pKa of the following equilibrium:

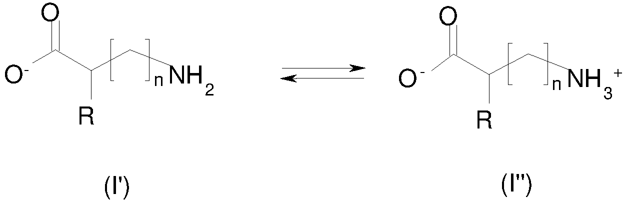

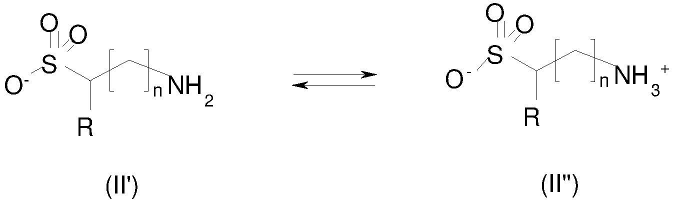

5. The process according to any one of claims 1 to 4, wherein the (a) compound is selected from the group consisting of alkylaminosulfonic acids such as 2-(cyclohexylamino)ethanesulfonic acid; amino acids such as glycine, alanine, glutamic acid, aspartic acid, phenylalanine, β-alanine, isoleucine, leucine, proline, glutamine, serine, threonine, valine, tryptophan, and tyrosine; oligomers of amino acids such as glycylglycine; aminosulfonic acids such as taurine; and mixtures thereof.

6. The process according to any one of claims 1 to 5, wherein the (a) compound is selected from the group consisting of 2-(cyclohexylamino)ethanesulfonic acid, glycine, alanine, taurine, and mixtures thereof.

7. The process according to any one of claims 1 to 6, wherein an amount of the (a) compound in the composition is from 1% to 20% by weight, preferably from 3% to 15% by weight, and more preferably from 5% to 10% by weight, relative to the total weight of the composition.

8. The process according to any one of claims 1 to 7, wherein an amount of the (b) monoethanolamine in the composition is from 0.1% to 15% by weight, preferably from 0.5% to 10% by weight, and more preferably from 1% to 5% by weight, relative to the total weight of the composition.

9. The process according to any one of claims 1 to 8, wherein the composition for straightening keratin fibers comprises (c) at least one diol selected from C₄₋₅ diols.

10. The process according to any one of claims 1 to 9, wherein the composition for straightening keratin fibers comprises (d) at least one organic salt of alkaline earth metal.

11. The process according to any one of claims 1 to 10, wherein the composition does not comprise any ammonia or a thiol compound, or comprises less than 1%, preferably less than 0.5%, and more preferably less than 0.1% by weight of ammonia or a thiol compound, relative to the total weight of the composition.

12. The process according to any one of claims 1 to 11, wherein the composition does not comprise any reducing agent or oxidizing agent, or comprises less than 1%, preferably less than 0.5%, and more preferably less than 0.1% by weight of a reducing agent or an oxidizing agent, relative to the total weight of the composition.

## Patentansprüche

1. Verfahren zum Glätten von Keratinfasern, vorzugsweise Haare, umfassend die folgenden Schritte:
(i) Auftragen, auf die Keratinfasern, einer Zusammensetzung zum Glätten von Keratinfasern;
(ii) Umhüllen der Keratinfasern, auf die die Zusammensetzung aufgetragen wird, mit mindestens einer Umhüllungseinrichtung, um die Keratinfasern feucht zu halten;
(iii) Glätten der feuchten Keratinfasern mit einem Heizeisen bei einer Temperatur von mehr als 100 °C, vorzugsweise mehr als 100 °C und weniger als 210 °C, und vorzugsweise von 110 °C bis 200 °C;
(iv) Enthüllen der Keratinfasern; und
(v) optional Spülen und/oder Trocknen der Keratinfasern,
wobei
die Zusammensetzung zum Glätten von Keratinfasern Folgendes umfasst
(a) mindestens eine Verbindung, ausgewählt aus Alkylaminosulfonsäuren und Verbindungen der folgenden Formeln (I) und (II): 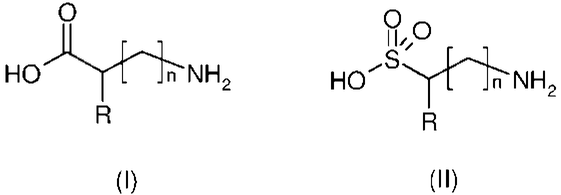 wobei in denen in den Formeln (I) und (II)
- R ein Wasserstoffatom oder eine lineare oder verzweigte, vorzugsweise lineare C₁-C₅-Alkylgruppe darstellt, wobei die Alkylgruppe optional mit mindestens einer Gruppe substituiert ist, die ausgewählt ist aus einer Hydroxylgruppe, einer Aminogruppe, einer Carboxamidogruppe, einer aromatischen C₆-C₁₈-Gruppe, einer heterocyclischen Gruppe, -C(O)-OH, -S(O)₂-OH, -C(O)-O⁻M⁺, -S(O)₂-O⁻M⁺ und Gemischen davon, wobei M⁺ ein kationisches Gegenion, wie beispielsweise ein Alkalimetall, Erdalkalimetall oder Ammonium, darstellt, und
- n 0 oder 1 ist,
und
b) Monoethanolamin,
und
die Zusammensetzung zum Glätten von Keratinfasern einen pH von 7,5 bis 12,0, vorzugsweise von 8,0 bis 11,5 und bevorzugter von 8,5 bis 11,0 aufweist.

2. Verfahren nach Anspruch 1, wobei die Umhüllungseinrichtung hitzebeständig ist, vorzugsweise gegen eine Temperatur von mehr als 100 °C, vorzugsweise 110 °C oder mehr und noch bevorzugter 120 °C oder mehr.

3. Verfahren nach Anspruch 1 oder 2, wobei die Umhüllungseinrichtung mindestens einen okklusiven Raum bildet, der die Keratinfasern beinhaltet.

4. Verfahren nach einem der Ansprüche 1 bis 3, wobei der pH der Zusammensetzung zum Glätten von Keratinfasern innerhalb von ±2 bezogen auf einen pH-Wert ist, der dem pKa-Wert des folgenden Gleichgewichts entspricht:

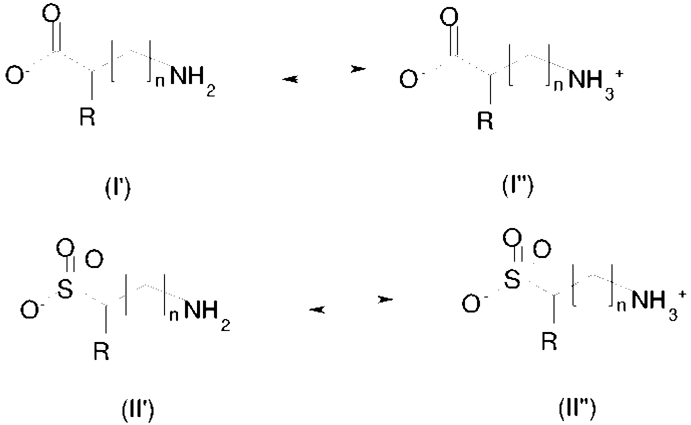

5. Verfahren nach einem der Ansprüche 1 bis 4, wobei die (a)-Verbindung ausgewählt ist aus der Gruppe, bestehend aus Alkylaminosulfonsäuren, wie beispielsweise 2-(Cyclohexylamino)ethansulfonsäure; Aminosäuren, wie beispielsweise Glycin, Alanin, Glutaminsäure, Asparaginsäure, Phenylalanin, β-Alanin, Isoleucin, Leucin, Prolin, Glutamin, Serin, Threonin, Valin, Tryptophan und Tyrosin; Oligomeren von Aminosäuren, wie beispielsweise Glycylglycin; Aminosulfonsäuren, wie beispielsweise Taurin; und Gemischen davon.

6. Verfahren nach einem der Ansprüche 1 bis 5, wobei die (a)-Verbindung ausgewählt ist aus der Gruppe bestehend aus 2-(Cyclohexylamino)ethansulfonsäure, Glycin, Alanin, Taurin und Gemischen davon.

7. Verfahren nach einem der Ansprüche 1 bis 6, wobei eine Menge der (a)-Verbindung in der Zusammensetzung von 1 bis 20 Gewichts-%, vorzugsweise von 3 bis 15 Gewichts-% und bevorzugter von 5 bis 10 Gewichts-%, bezogen auf das Gesamtgewicht der Zusammensetzung, ist.

8. Verfahren nach einem der Ansprüche 1 bis 7, wobei eine Menge des (b) Monoethanolamins in der Zusammensetzung von 0.1 bis 15 Gewichts-%, vorzugsweise von 0.5 bis 10 Gewichts-% und bevorzugter von 1 bis 5 Gewichts-%, bezogen auf das Gesamtgewicht der Zusammensetzung, ist.

9. Verfahren nach einem der Ansprüche 1 bis 8, wobei die Zusammensetzung zum Glätten von Keratinfasern (c) mindestens ein Diol umfasst, ausgewählt aus C₄₋₅-Diolen.

10. Verfahren nach einem der Ansprüche 1 bis 9, wobei die Zusammensetzung zum Glätten von Keratinfasern (d) mindestens ein organisches Salz von Erdalkalimetall umfasst.

11. Verfahren nach einem der Ansprüche 1 bis 10, wobei die Zusammensetzung kein Ammoniak oder eine Thiolverbindung enthält oder weniger als 1 Gewichts-%, vorzugsweise weniger als 0,5 Gewichts-% und bevorzugter weniger als 0,1 Gewichts-% Ammoniak oder eine Thiolverbindung, bezogen auf das Gesamtgewicht der Zusammensetzung, umfasst.

12. Verfahren nach einem der Ansprüche 1 bis 11, wobei die Zusammensetzung kein Reduktionsmittel oder Oxidationsmittel umfasst oder weniger als 1 Gewichts-%, vorzugsweise weniger als 0,5 Gewichts-% und bevorzugter weniger als 0,1 Gewichts-% eines Reduktionsmittels oder eines Oxidationsmittels, bezogen auf das Gesamtgewicht der Zusammensetzung, umfasst.

## Revendications

1. Procédé de lissage de fibres kératiniques, de préférence des cheveux, comprenant les étapes consistant à :
(i) appliquer sur les fibres kératiniques une composition pour lissage de fibres kératiniques ;
(ii) envelopper les fibres kératiniques, sur lesquelles la composition est appliquée, à l'aide d'au moins un moyen d'enveloppement afin de maintenir les fibres kératiniques humides ;
(iii) lisser les fibres kératiniques humides à l'aide d'un fer à lisser à une température supérieure à 100 °C, de préférence supérieure à 100 °C et inférieure à 210 °C, et de préférence de 110 °C à 200 °C ;
(iv) désenvelopper les fibres kératiniques ; et
(v) facultativement rincer et/ou sécher les fibres kératiniques,
dans lequel
la composition pour lissage de fibres kératiniques comprend
(a) au moins un composé choisi parmi les acides alkylamino-sulfoniques et des composés de formules (I) et (II) suivantes : 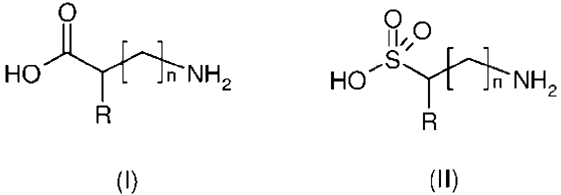 dans lequel, dans les formules (I) et (II)
- R représente un atome d'hydrogène ou un groupe alkyle linéaire ou ramifié, de préférence linéaire, un groupe alkyle en C₁-C₅, ledit groupe alkyle étant facultativement substitué par au moins un groupe choisi parmi un groupe hydroxyle, un groupe amino, un groupe carboxamido, un groupe aromatique en C₆-C₁₈, un groupe hétérocyclique, -C(O)-OH, -S(O)₂-OH, -C(O)-O⁻M⁺, -S(O)₂-O⁻M⁺, et les mélanges de ceux-ci, avec M⁺ représentant un contre-ion cationique tel qu'un métal alcalin, un métal alcalino-terreux ou un ammonium, et
- n est 0 ou 1,
et
b) une monoéthanolamine,
et
la composition pour lissage de fibres kératiniques a un pH de 7,5 à 12,0, de préférence de 8,0 à 11,5, et plus préférentiellement de 8,5 à 11,0.

2. Procédé selon la revendication 1, dans lequel le moyen d'enveloppement est résistant à la chaleur, de préférence à une température supérieure à 100 °C, plus préférentiellement à 110 °C ou plus, et encore plus préférentiellement à 120 °C ou plus.

3. Procédé selon la revendication 1 ou 2, dans lequel le moyen d'enveloppement forme au moins un espace occlusif incluant les fibres kératiniques.

4. Procédé selon l'une quelconque des revendications 1 à 3, dans lequel le pH de la composition de lissage de fibres kératiniques se situe à ±2 par rapport à un pH qui est égal au pKa de l'équilibre suivant :

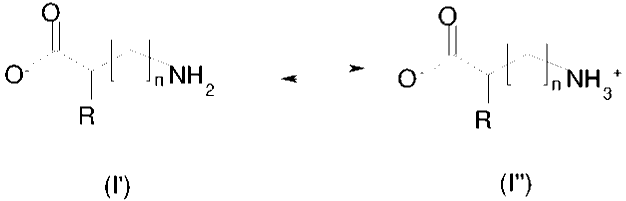

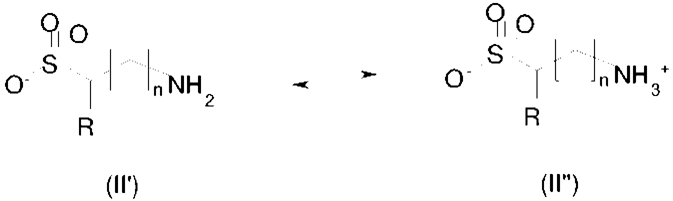

5. Procédé selon l'une quelconque des revendications 1 à 4, dans lequel le composé (a) est choisi dans le groupe constitué par les acides alkylaminosulfoniques tels que l'acide 2-(cyclohexylamino)éthanesulfonique ; les acides aminés tels que la glycine, l'alanine, l'acide glutamique, l'acide aspartique, la phénylalanine, la β-alanine, l'isoleucine, la leucine, la proline, la glutamine, la sérine, la thréonine, la valine, le tryptophane et la tyrosine ; les oligomères d'acides aminés tels que la glycylglycine ; les acides aminosulfoniques tels que la taurine ; et leurs mélanges.

6. Procédé selon l'une quelconque des revendications 1 à 5, dans lequel le composé (a) est choisi dans le groupe constitué par l'acide 2-(cyclohexylamino)éthanesulfonique, la glycine, l'alanine, la taurine et leurs mélanges.

7. Procédé selon l'une quelconque des revendications 1 à 6, dans lequel une quantité du composé (a) dans la composition est de 1 % à 20 % en poids, de préférence de 3 % à 15 % en poids, plus préférentiellement de 5 % à 10 % en poids, par rapport au poids total de la composition.

8. Procédé selon l'une quelconque des revendications 1 à 7, dans lequel une quantité de la monoéthanolamine (b) dans la composition est de 0,1 % à 15 % en poids, préférentiellement de 0,5 % à 10 % en poids, plus préférentiellement de 1 % à 5 % en poids, par rapport au poids total de la composition.

9. Procédé selon l'une quelconque des revendications 1 à 8, dans lequel la composition de lissage de fibres kératiniques comprend (c) au moins un diol choisi parmi les diols en C₄₋₅.

10. Procédé selon l'une quelconque des revendications 1 à 9, dans lequel la composition de lissage de fibres kératiniques comprend (d) au moins un sel organique de métal alcalino-terreux.

11. Procédé selon l'une quelconque des revendications 1 à 10, dans lequel la composition ne comprend pas d'ammoniac ou de composé thiol, ou comprend moins de 1 %, de préférence moins de 0,5 %, et plus préférentiellement moins de 0,1 % en poids d'ammoniac ou d'un composé thiol, par rapport au poids total de la composition.

12. Procédé selon l'une quelconque des revendications 1 à 11, dans lequel la composition ne comprend pas d'agent réducteur ou d'agent oxydant, ou comprend moins de 1 %, de préférence moins de 0,5 %, et plus préférentiellement moins de 0,1 % en poids d'un agent réducteur ou d'un agent oxydant, par rapport au poids total de la composition.
